# EUROPEAN PATENT APPLICATION

(11) **EP 4 796 566 A1**
(43) Date of publication of application: **26.08.2026**
(21) Application number: 24879757.3
(22) Date of filing: 16.10.2024
(51) Int. Cl.: C07K 19/00, C07K 7/50, C07K 14/015, C07K 16/28, C12N 15/864

(54) **MOLECULE CAPABLE OF SIMULTANEOUSLY BINDING TO AAV CAPSID AND TARGET RECEPTOR**

(30) Priority: 17.10.2023 JP 2023179190
(71) Applicant: The University of Osaka, Suita-shi, Osaka 5650871 (JP); The University Of Tokyo, Tokyo 113-8654 (JP)
(72) Inventor: TAKAGI, Junichi, Osaka 5650871 (JP); MIHARA, Emiko, Osaka 5650871 (JP); SUGA, Hiroaki, Tokyo 1138654 (JP); ANANANUCHATKUL, Teerapat, Tokyo 1138654 (JP)
(74) Representative: Engelhard, Markus
(86) International application number: PCT/JP2024/036910
(87) International publication number: WO 2025/084336

(57) **Abstract**

The present invention discloses, as one embodiment, a bivalent molecule capable of binding to both native AAV capsid and molecular target. As another embodiment, a method for obtaining high gene transfer efficiency in a receptor-dependent manner is also disclosed which uses, as a vector, a complex of this molecule and native AAV capsid formed using this molecule as an engager. These embodiments contribute to simultaneously achieving two purposes of using native AAV capsid free of any modification and dramatically increasing the infectivity to target cells. These embodiments are useful, for example, in the fields of gene therapy and the like.

## Description

### [Technical Field]

The present invention relates to a method for imparting specificity for a target molecule to an adeno-associated virus (hereinafter also denoted as "AAV"), and provides, as one embodiment of the invention, a bivalent molecule capable of binding to both the capsid of a native adeno-associated virus and a molecular target. The present invention is useful, for example, in the field of gene therapy and the like.

### [Background Art]

Viruses that deliver their own genes to host cells have been used in the field of gene therapy as a means to deliver therapeutic genes to patients. However, not all viruses are suitable for this purpose due to the pathogenicity and immunogenicity. AAV is one of the most generally-used viruses because it is non-pathogenic to humans and has a superior safety profile (Non Patent Literature 1).

AAV transduction of cells is initiated by attachment of virus to the cell via cell surface glycan moiety (hereafter referred to as attachment receptor). The amino acid sequence of the capsid surface loop involved in the binding of attachment receptors differ among different serotypes, due to which the chemical nature of a particular AAV attachment receptor depends on the serotype. For example, AAV serotype 2 (AAV2) binds to heparan sulfate proteoglycan (HSPG), AAV1 to sialic acid, and AAV9 to terminal galactose sugar chain. After attachment, AAV binds to a second class of receptor (generally referred to as internalization receptor), which initiates viral internalization via endocytosis. The gene product KIAA0319L (later renamed AAVR) was discovered as a universal coreceptor for internalization of most AAV serotypes, with a few exceptions including AAV4 (Non Patent Literature 2). AAVR is localized in the intracellular trans-Golgi network (TGN) under steady-state conditions, and cells lacking this are unable to be infected by AAV. Therefore, AAVR-mediated retrograde transport is considered to be essential for AAV transduction. AAVR is also recognized by a protruding loop on the surface of the AAV capsid, and its interaction pattern has been elucidated by cryo-electron microscopy.

Among viral vectors, AAV has recently emerged as a promising candidate for gene therapy. This is evidenced by the FDA approval of two drugs, Zolgensma (using AAV9 as the capsid) for spinal muscular atrophy and HemGenix (using AAV5 as the capsid) for hemophilia, in 2020 and 2022, respectively. AAV has relatively low immunogenicity, can be introduced into a wide range of cells including both dividing and non-dividing cells, and has the property of long-term expression derived from stable episomes. Thus, AAV has become a suitable vector for gene therapy.

However, despite these successful drug developments, current AAV vectors have many problems, and the need for fundamental improvement is demanded. The main issues are the low infection specificity and low transduction efficiency of AAV, which necessitate high-dose administration to achieve therapeutic effects in target organs (Non Patent Literature 3). Administration of such high vector doses of AAV can cause severe side effects, and in fact, multiple deaths due to liver dysfunction occurred in clinical trials of X-linked myotubular myopathy (Non Patent Literature 4). The need for high-dose administration also necessitates the production of large quantities of medical viral vectors, and poses a hurdle to the medical application of AAV vectors in terms of production cost, apart from safety. Therefore, improving specificity and transduction efficiency to reduce vector doses is desired. For this end, a method that has become increasingly performed in recent years is modification of the capsid itself, which is the primary determinant of AAV tropism and transduction properties (Non Patent Literature 3).

A general approach to capsid modification is to first prepare a library of AAV capsid variants through random mutagenesis, capsid shuffling, or short random peptide insertion, and then administer this to animals to select candidates with the desired tropism or properties. However, this conventional approach requires not only the genetic engineering and production to create millions of different viruses, but also the enormous time and effort for identification and evaluation thereof. Moreover, even if promising mutant capsids are obtained through such efforts, their in vivo characteristics depend on various factors, and they are often unable to be applied to animal species or serotypes other than those used for screening (Non Patent Literature 5). Furthermore, a problem with variants obtained by in vivo selection starting from a randomized capsid library is that the molecular mechanisms and responsible receptors behind their properties, such as infection specificity thereof and improved transduction efficiency, often remain unclear.

In contrast to the above-mentioned approach of stochastically obtaining desirable mutant viruses through screening from randomized libraries, rational design approach utilizes existing knowledge of intermolecular interactions to highly predict and modify the properties of mutant viruses from the outset. A notable example of successful rational AAV design is the insertion or fusion of small binder proteins for specific cellular receptors, such as nanobody, DARPin, and the like, into the capsid (Non Patent Literatures 6 and 7). However, this strategy has the drawback that the prior acquisition of such specialized binder molecule is required. More importantly, this method requires insertion of considerably large protein domains (nanobody and DARPin are both about 15 kDa) into the capsid subunit, which poses an extremely high risk of compromising the structural and functional integrity of the capsid. In fact, it is known that the insertion of even a very short random peptide (7 amino acids) significantly alters the chemical structure of the capsid surface, making most of the obtained variants unavailable for recombinant expression. In other words, it has been pointed out that whether one starts from a random library using directed evolution or utilizes rational design, any modification of a naturally occurring AAV serotype, even slightly, requires detailed verification of its recombinant production efficiency, pharmacokinetics, and unexpected human toxicity before it can be applied to actual gene therapy (Non Patent Literature 8).

As mentioned above, modifications of various AAV capsids have been attempted to overcome the drawbacks of existing gene therapy AAV vector, namely, "low target specificity" and "low transduction efficiency". However, most of these modifications aim to retarget AAV to specific membrane proteins by conferring the ability to interact with novel molecules that do not serve as infection receptors for natural AAV. However, retargeting is difficult with this alone. This is because even after modification, AAV retains its ability to infect via its "normal" attachment receptor, and therefore continues to transduce cells in organs and tissues other than the therapeutic target, resulting in a limited increase in actual infection specificity. Therefore, some retargeting strategies involve eliminating the native tropism of the original viral vector and conferring new molecular specificity.

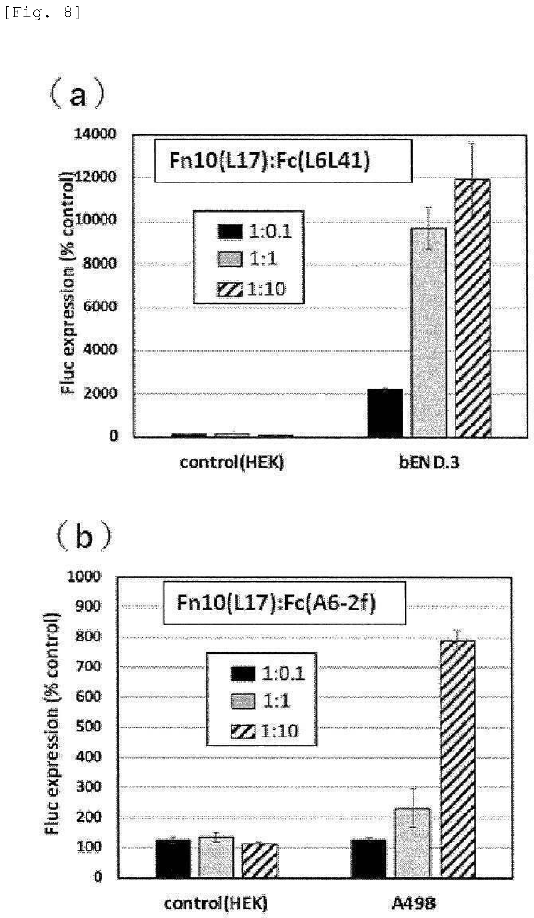

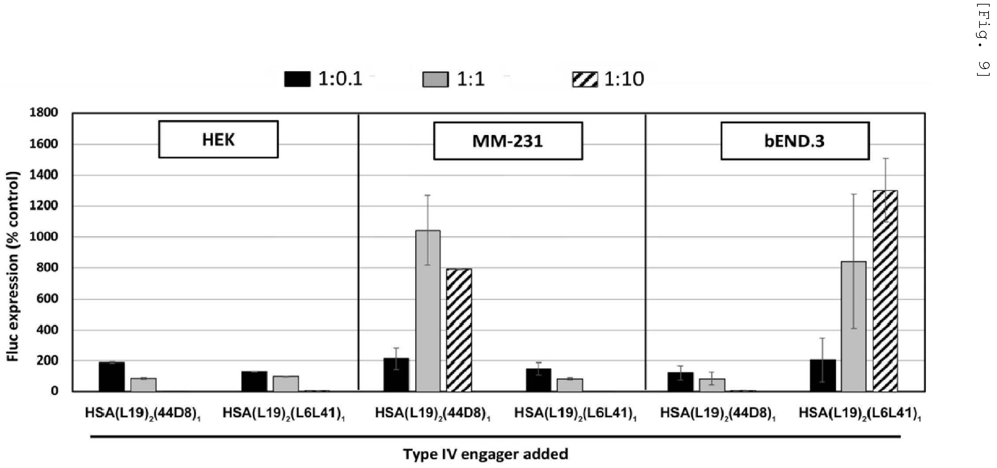

For example, Patent Literatures 1 and 2 describe a method for obtaining a highly efficiently retargeted modified virus by replacing a key amino acid region on the native AAV capsid subunit involved in binding to a cellular attachment receptor with an artificial tag peptide sequence, thereby neutralizing the native cell tropism and simultaneously conferring new receptor-binding properties to molecules (e.g., antibodies) that bind to the tag peptide sequence. However, with this method, the binding ability of the attachment and internalization receptors is significantly weakened when the capsid is modified to eliminate the native tropism. Therefore, even after acquiring targeting, the gene transfer ability remains at the same level or even lower than that of the unmodified wild-type virus (e.g., Fig. 8B, Fig. 9-2, etc. in Patent Literature 1). Furthermore, these strategies still suffer from the problems inherent to modified capsid, as mentioned above.

A method for rationally designing and converting viral targeting has also been devised, which uses molecules known as "adapters" (Non Patent Literature 9). An "adapter" presents a foreign molecule capable of binding to a target receptor on a virus, thereby bringing the virus into close proximity with specific cells and altering infection specificity. Several adapter strategies have been proposed for adenovirus (Ad) and AAV, but most involve capsid modification (Patent Literatures 1 and 2, and Non Patent Literatures 10 and 11), with few examples using natural viral capsids. The only reported techniques that do not involve capsid modification are those using soluble viral receptors (CAR) (Non Patent Literature 12) and artificial binder proteins called DARPin (Non Patent Literature 13) for Ad, and those using antibodies against Cap subunits for AAV (Non Patent Literature 14). However, these adapters cover the sites (loops protruding from the surface) where the capsid binds to attachment receptors or internalization receptors. Therefore, even if the ability to bind to a new target receptor is acquired through adapter binding, the gene transfer efficiency of the virus as a whole generally cannot be significantly increased compared to the original virus (without addition of adapter).

### [Citation List]

### [Patent Literature]

[Patent Literature 1]
   JP 2020-525020 A
[Patent Literature 2]
   JP 2020-529196 A

### [Non Patent Literature]

[Non Patent Literature 1]
   Zhao et al, Bioeng Transl Med. 2021 Oct 20; 7(1): e10258
[Non Patent Literature 2]
   Pillay et al, Nature 530, 108-112 (2016)
[Non Patent Literature 3]
   Rodriguez-Marquez et al, Expert Opin Biol Ther. 2021 Jun; 21(6):749-766
[Non Patent Literature 4]
   Nat Biotechnol 38, 910 (2020)
[Non Patent Literature 5]
   Davidsson et al, Proc Natl Acad Sci U S A. 2019 Dec 26; 116(52):27053-27062
[Non Patent Literature 6]
   Eichhoff et al, Mol Ther Methods Clin Dev. 2019 Sep 16; 15: 211-220
[Non Patent Literature 7]
   Munch et al, Nat Commun. 2015 Feb 10; 6: 6246
[Non Patent Literature 8]
   Margaret et al, ACS Nano, 2020, 14, 14262-83.
[Non Patent Literature 9]
   Reinhard et al. Nature Rev. Genetics, 2007, 8, 573-587
[Non Patent Literature 10]
   Parrott et al, Mol. Ther. 8, 688-700 (2003)
[Non Patent Literature 11]
   Gigout et al, Mol. Ther. 11, 856-865 (2005)
[Non Patent Literature 12]
   Pereboev et al, Mol. Ther. 9, 712-720 (2004)
[Non Patent Literature 13]
   Dreier et al, Proc Natl Acad Sci USA. 2013 Mar 5; 110(10): E869-77
[Non Patent Literature 14]
   Bartlett et al, Nature Biotechnol. 17, 181-186 (1999)

### [Summary of Invention]

### [Technical Problem]

As described above, no technology exists that simultaneously achieves the two purposes of (1) using a completely unmodified, natural AAV capsid and (2) dramatically increasing infectivity in target cells. Therefore, a new technology that achieves the both has been awaited.

### [Solution to Problem]

As one of the embodiments of the present invention, the present specification describes an AAV retargeting strategy that uses a completely unmodified, naturally occurring AAV capsid serotype to resolve the problems unavoidable with previously engineered modified capsids. More particularly, a method is disclosed that uses a novel bivalent molecule that can bind to both the native AAV capsid and a molecular target, in which a complex of this molecule and the native AAV capsid as a vector is allowed to act on cells, whereby gene transfer efficiency that is dramatically higher than when native AAV alone is used as a vector is achieved in a receptor-dependent manner.

Specific embodiments of the AAV retargeting strategy described above include, but are not limited to, the following.
[1] A molecule comprising a module (A) capable of binding to a capsid of AAV (adeno-associated virus) ("AAV capsid-binding property") and a module (B) capable of binding to a target receptor ("target receptor-binding property"), and capable of simultaneously binding to the capsid of AAV and the target receptor, wherein
the module (A) is 1) an AAV capsid-binding peptide, or 2) a scaffold protein having a structure in which an AAV capsid-binding peptide is grafted (more specifically, a structure in which an AAV capsid-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein) (hereinafter also denoted as "the molecule").
[2] The molecule of the above-mentioned [1], wherein the module (B) is 1) an anti-target receptor antibody, 2) a target receptor-binding peptide, or 3) a scaffold protein having a structure in which a target receptor-binding peptide is grafted (more specifically, a structure in which a target receptor-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein).
[3] The molecule of the above-mentioned [1] or [2], which is an anti-target receptor antibody having a structure in which an AAV capsid-binding peptide is grafted.
[4] The molecule of the above-mentioned [1] or [2], which is a protein to which a scaffold protein having a structure in which an AAV capsid-binding peptide is grafted and an anti-target receptor antibody are linked.
[5] The molecule of the above-mentioned [1] or [2], which is a protein to which a scaffold protein having a structure in which an AAV capsid-binding peptide is grafted and a scaffold protein having a structure in which a target receptor-binding peptide is grafted are linked.
[6] The molecule of the above-mentioned [1] or [2], which is a scaffold protein having a structure in which an AAV capsid-binding peptide and a target receptor-binding peptide are grafted.
[7] The molecule of the above-mentioned [1], [2] or [3], which is an anti-target receptor antibody having an amino acid sequence incorporating all or part of an AAV capsid-binding peptide sequence,
which antibody is composed of an anti-target receptor antibody having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.
[8] The molecule of the above-mentioned [1], [2] or [4], which is a protein to which
an anti-target receptor antibody and an AAV capsid-binding scaffold protein having an amino acid sequence incorporating all or part of an AAV capsid-binding peptide sequence are linked,
which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.

[9] The molecule of the above-mentioned [1], [2] or [5], which is a protein to which
an AAV capsid-binding scaffold protein having an amino acid sequence incorporating all or part of an AAV capsid-binding peptide sequence, which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and
a target receptor-binding scaffold protein having an amino acid sequence incorporating all or part of a target receptor-binding peptide sequence, which target receptor-binding scaffold protein is composed of a scaffold protein having at least one loop structure wherein all or part of an amino acid sequence constituting the at least one loop structure is replaced with all or part of the target receptor-binding peptide sequence, or all or part of the target receptor-binding peptide sequence is inserted into an amino acid sequence constituting the at least one loop structure
are linked.

[10] The molecule of the above-mentioned [1], [2] or [6], which is a protein having an amino acid sequence incorporating all or part of each of an AAV capsid-binding peptide sequence and a target receptor-binding peptide sequence, which protein is composed of a scaffold protein having two or more loop structures
wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and wherein all or part of an amino acid sequence constituting at least one loop structure different from the above-mentioned loop structure is replaced with all or part of the target receptor-binding peptide sequence, or all or part of the target receptor-binding peptide sequence is inserted into an amino acid sequence constituting the at least one loop structure different from the above-mentioned loop structure.
[11] The molecule of the above-mentioned [1] of [2], which is a protein in which a biotinylated AAV capsid-binding peptide and a biotinylated target receptor-binding peptide are linked via a protein having two or more biotin-binding sites.
[12] The molecule of the above-mentioned [1] or [2], which is a protein in which a biotinylated AAV capsid-binding peptide and a biotinylated anti-target receptor antibody are linked via a protein having two or more biotin-binding sites.
[13] The molecule of any of the above-mentioned [1] to [12], wherein the AAV capsid-binding peptide is a full sequence or a partial sequence constituting a cyclic peptide ("AAV capsid-binding cyclic peptide") that contains an AAV capsid-binding amino acid sequence in the peptide sequence.
[14] The molecule of the above-mentioned [13], wherein the AAV capsid-binding cyclic peptide is a cyclic peptide having a cyclic structure formed via a crosslinking bond connecting one amino acid to another amino acid in the peptide sequence.
[15] The molecule of the above-mentioned [13] or [14], wherein the AAV capsid-binding cyclic peptide has a cyclic structure comprising 4 to 30 amino acids.
[16] The molecule of the above-mentioned [14], wherein the crosslinked structure comprises a thioether bond or a disulfide bond.
[17] The molecule of any of the above-mentioned [13] to [16], wherein the AAV capsid-binding cyclic peptide has a cyclic structure formed via a crosslinking bond connecting an amino acid having a Functional Group 1 ("ring-forming amino acid 1") shown in Functional Group groups (A) to (E) in the following Table 1, with an amino acid having the corresponding Functional Group 2 ("ring-forming amino acid 2").

**[Table 1]**

| | Functional Group 1 | Functional Group 2 |
|---|---|---|
| (A) | 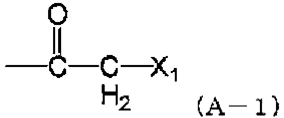 | HS- (A-2) |
| (B) | -C≡C-H (B-1) | N₃- (B-2) |
| (C) | -Ar-CH₂NH₂ (C-1) | 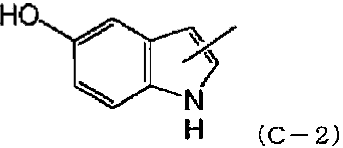 |
| (D) | -C≡C-CH₂-X₁ (D- 1) | HS- (D-2) |
| (E) | -Ar-CH₂-X₁ (E - 1) | HS- (E-2) |

| | | |
|---|---|---|
| wherein X₁ is a leaving group, and Ar is an aromatic ring optionally having substituent(s). | | |

[18] The molecule of the above-mentioned [17], wherein the ring-forming amino acid 1 is an amino acid having a Functional Group (A-1) described in the aforementioned Table 1, the ring-forming amino acid 2 is an amino acid having the corresponding Functional Group (A-2), and the cyclic structure comprises an - N-CO-CH₂-S- structure formed between the both amino acids.
[19] The molecule of the above-mentioned [17], wherein N in the -N-CO-CH₂-S- structure is derived from an amino group of the ring-forming amino acid 1 selected from alanine, tyrosine, phenylalanine, tryptophan, ornithine, lysine, diaminopropionic acid, and diaminobutyric acid.
[20] The molecule of the above-mentioned [17], wherein S in the -N-CO-CH₂-S- structure is derived from a thiol group of the ring-forming amino acid 2 selected from cysteine, homo cysteine, mercaptonorvaline, mercaptonorleucine, 2-amino-7-mercaptoheptanoic acid, and 2-amino-8-mercaptooctanoic acid.
[21] The molecule of any of the above-mentioned [13] to [20], wherein the AAV capsid-binding cyclic peptide is a cyclic peptide comprising, in the peptide sequence, an ("AAV9 capsid-binding") amino acid sequence capable of binding to a capsid of AAV9 (adeno-associated virus serotype 9) ("AAV9 capsid-binding cyclic peptide").
[22] The molecule of the above-mentioned [21], wherein the AAV9 capsid-binding cyclic peptide has a cyclic structure formed via
1) a crosslinking bond connecting one amino acid located at one end of the AAV9 capsid-binding amino acid sequence or located outside that end to another amino acid located at the other end of the AAV9 capsid-binding amino acid sequence or located outside that end, or
2) a crosslinking bond connecting one amino acid located at one end of the AAV9 capsid-binding amino acid sequence or located outside that end to another amino acid located within the AAV9 capsid-binding amino acid sequence.

[23] The molecule of the above-mentioned [22], wherein the AAV9 capsid-binding amino acid sequence is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn.

[24] The molecule of the above-mentioned [23], wherein the core motif sequence (II) is
(1) the amino acid sequence shown in Tyr-Ser-Lys-Ser-Val-Cys-Leu-Ala-Gln-Asp (SEQ ID NO: 2), or
   an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.
[25] The molecule of any of the above-mentioned [21] to [24], wherein the active structure of the AAV9 capsid-binding cyclic peptide is selected from cyclic peptides represented by the following formula (Ia), the formula (Ib), and the formula (Ic): 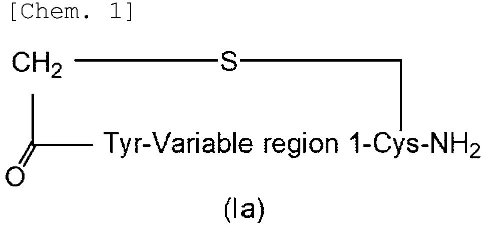
wherein Variable region 1 is an AAV9-binding amino acid sequence selected from the amino acid sequences listed in the following Table 2, and
S is S derived from the thiol group of Cys

**[Table 2]**

| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

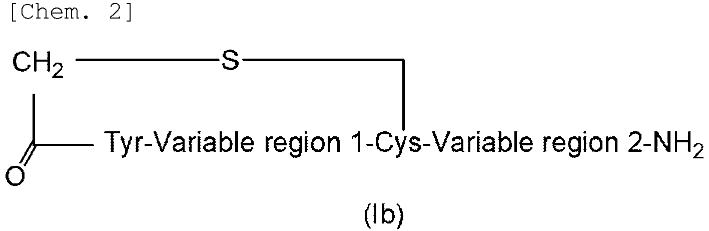

wherein Variable region 1 is an AAV9-binding amino acid sequence selected from the amino acid sequences listed in the following Table 3,
Variable region 2 is an AAV9-binding amino acid sequence selected from the amino acid sequences listed in the following Table 4, and
S is S derived from the thiol group of Cys

**[Table 3]**

| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |

wherein an amino acid sequence of Variable region 1 in Table 3 is preferably Ser Lys Ser Val (SKSV),

**[Table 4]**

| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

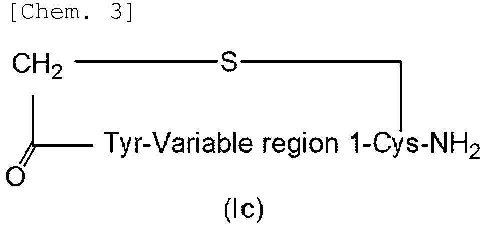

wherein Variable region 1 is an AAV9-binding amino acid sequence selected from the amino acid sequences listed in the following Table 5, and
S is S derived from the thiol group of Cys

**[Table 5]**

| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |

.
[26] The molecule of the above-mentioned [1] to [12], wherein the AAV capsid-binding peptide is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn, or
a sequence comprising the sequence.

[27] The molecule of the above-mentioned [26], wherein the core motif sequence (II) is
(1) the amino acid sequence shown in Tyr-Ser-Lys-Ser-Val-Cys-Leu-Ala-Gln-Asp (SEQ ID NO: 2), or
   an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.
[28] The molecule of any of the above-mentioned [1] to [12], wherein the AAV capsid-binding peptide is a full sequence or a partial sequence constituting the cyclic peptide represented by the following formula (Ia), the formula (Ib), or the formula (Ic) 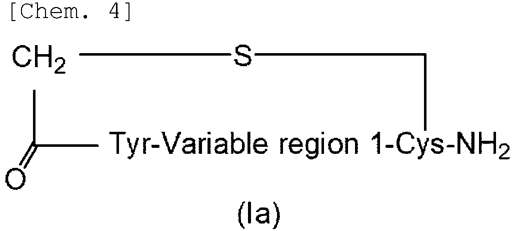
wherein Variable region 1 is an AAV9-binding amino acid sequence selected from the amino acid sequences listed in the following Table 6, and
S is S derived from the thiol group of Cys

**[Table 6]**

| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

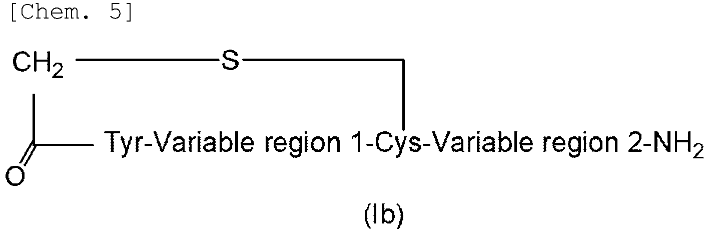

wherein Variable region 1 is an AAV9-binding amino acid sequence selected from the amino acid sequences listed in the following Table 7,
Variable region 2 is an AAV9-binding amino acid sequence selected from the amino acid sequences listed in the following Table 8, and
S is S derived from the thiol group of Cys

**[Table 7]**

| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |

wherein the amino acid sequence of Variable region 1 in Table 7 is preferably Ser Lys Ser Val (SKSV),

**[Table 8]**

| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

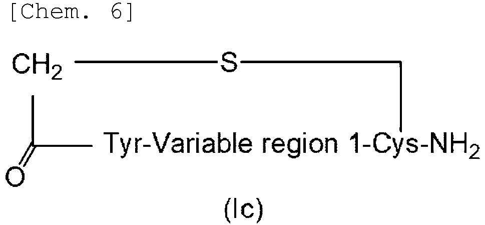

wherein Variable region 1 is an AAV9-binding amino acid sequence selected from the amino acid sequences listed in the following Table 9, and
S is S derived from the thiol group of Cys

**[Table 9]**

| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |

[29] The molecule of any of the above-mentioned [1], [2], [5], [6], and [9] to [11], wherein the target receptor-binding peptide is a full sequence or a partial sequence constituting a cyclic peptide comprising a target receptor-binding amino acid sequence in the peptide sequence ("target receptor-binding cyclic peptide").
[30] The molecule of the above-mentioned [29], wherein the target receptor-binding cyclic peptide is a cyclic peptide having a cyclic structure formed via a crosslinking bond connecting one amino acid to another amino acid in the peptide sequence.
[31] The molecule of any of the above-mentioned [1] to [30], that binds to AAV via module (A), for attenuating the nonspecific infectivity of AAV, while simultaneously increasing the specific infectivity for the receptor that is the target of module (B).
[32] An AAV vector comprising a complex of the molecule of any of the above-mentioned [1] to [30] and an AAV having a wild-type capsid, which complex is obtained by mixing the molecule and the AAV.
[33] The AAV vector of the above-mentioned [32], in which a foreign molecule is loaded onto the AAV.
[34] The AAV vector of the above-mentioned [32], for infecting cells that present a target receptor with AAV.
[35] The AAV vector of the above-mentioned [33], for introducing a foreign molecule into cells that present a target receptor.
[36] A method for infecting cells that present a target receptor of a subject with AAV, comprising administering the AAV vector of the above-mentioned [32] to the subject.
[37] A method for introducing a foreign molecule into cells that present a target receptor of a subject, comprising administering the AAV vector of the above-mentioned [33] to the subject.
[38] The AAV vector of the above-mentioned [32], used for infecting cells that present a target receptor with AAV.
[39] The AAV vector of the above-mentioned [33], used for introducing a foreign molecule into cells that present a target receptor.
[40] A cyclic peptide or a salt thereof, comprising an AAV capsid-binding amino acid sequence in the peptide sequence.
[41] A cyclic peptide or a salt thereof, comprising a target receptor-binding amino acid sequence in the peptide sequence.

### [Advantageous Effects of Invention]

One embodiment of the present invention provides a bivalent molecule capable of binding to both the capsid of a native adeno-associated virus and a molecular target. This molecule acts as an "engager" that attracts AAV to cells expressing a target receptor. Therefore, by allowing a complex of this molecule with a recombinant AAV containing a native AAV capsid to act on cells as a vector, receptor-dependent gene transfer efficiency strikingly higher than when native AAV alone is used as a vector is achieved. Furthermore, even though a native serotype of AAV is used, the complex with the above-mentioned molecule can suppress infection and gene transfer in cells that do not express the target receptor, and also affords a superior effect of inhibiting the binding of neutralizing antibodies to suppress neutralization of AAV by antibodies.

### [Brief Description of Drawings]

[Fig. 1]

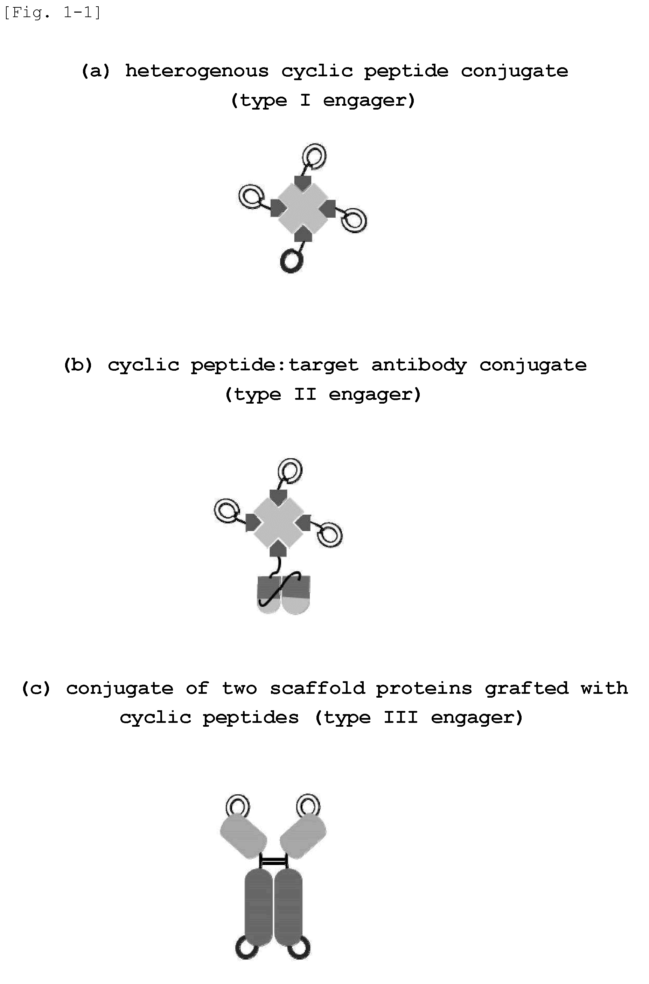

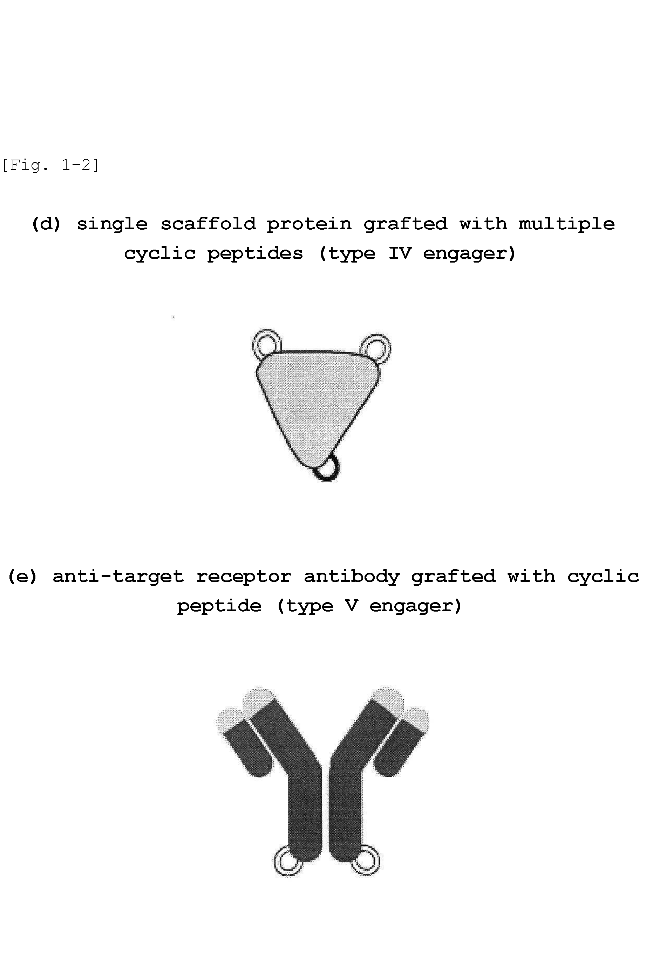

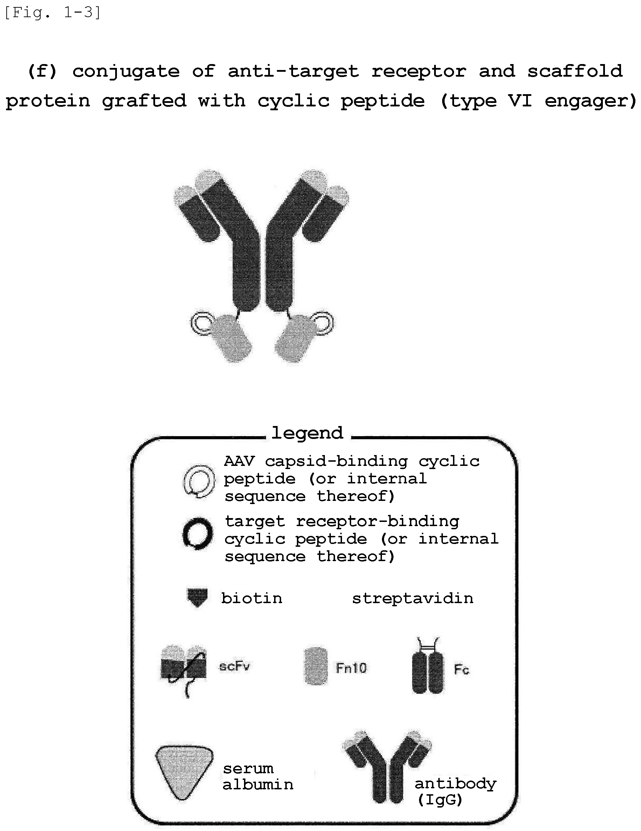

   Fig. 1 shows six representative embodiments of the above-mentioned molecule as a bivalent engager that binds to AAV and a target receptor, as described in the present specification.
[Fig. 2]

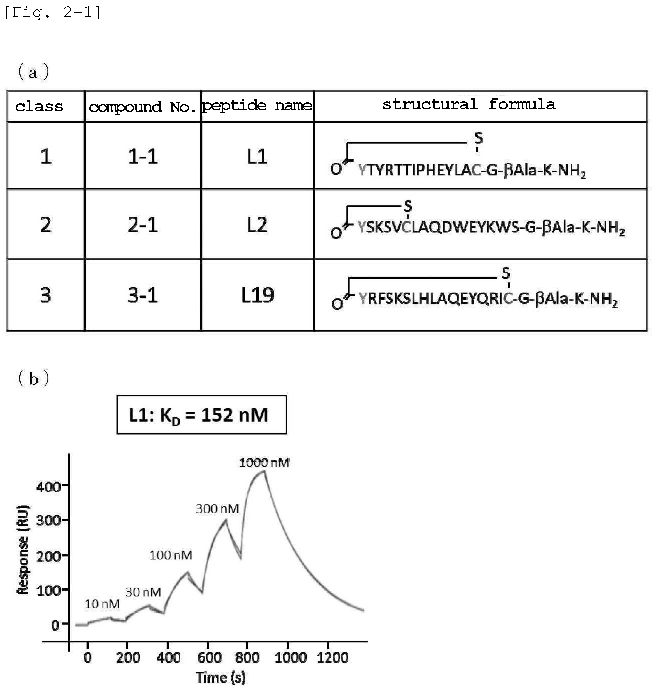

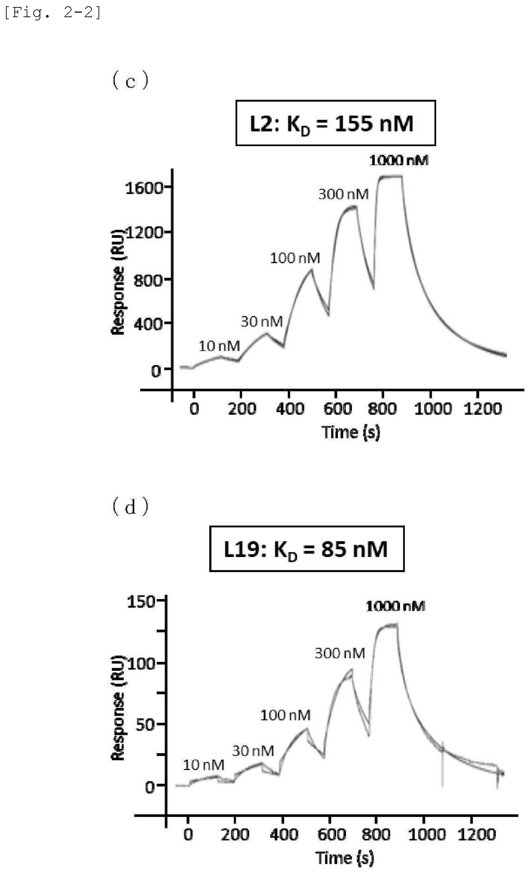

   Fig. 2a shows the names and structural formulas of the synthesized cyclic peptides selected as representatives from the three classes, and Figs. 2b to 2d show the analysis results of surface resonance plasmon (SPR) of the binding of the chemically synthesized cyclic peptides to the AAV9 capsid.
[Fig. 3]

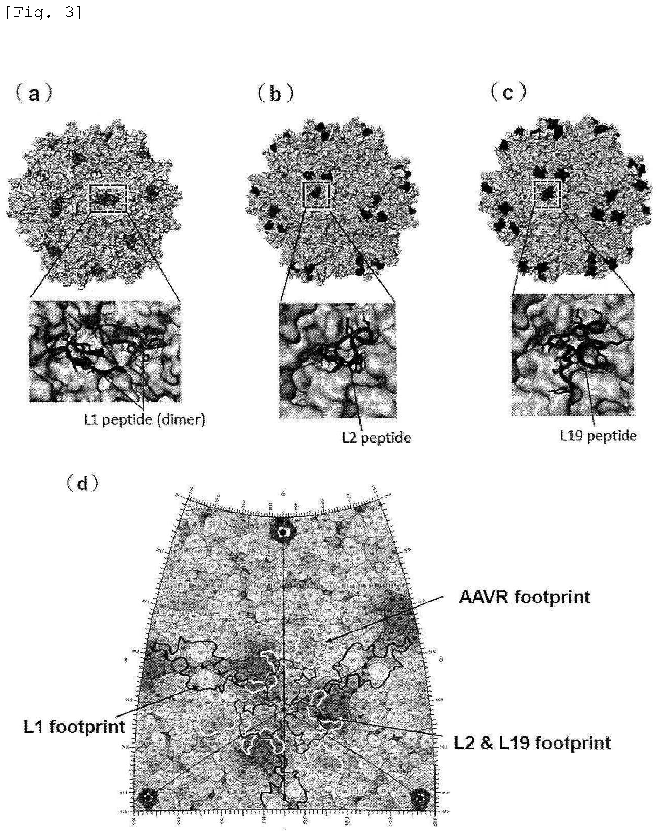

   Fig. 3 shows the AAV9-binding sites of cyclic peptides identified by cryo-electron microscopy imaging. Figs. 3a to 3c show the overall atomic model of the complexes of three AAV9-binding cyclic peptides and the AAV9 capsid in the upper panel, and the enlarged view of the binding site for one cyclic peptide molecule (a set of two molecules for L1 alone) in the lower panel. Fig. 3d shows a so-called "roadmap" in which the amino acid residues on the virus surface are projected onto a plane around the three-fold symmetry axis, with the solid lines indicating the binding regions (footprints) on the virus between the peptides and the infection receptor.
[Fig. 4]

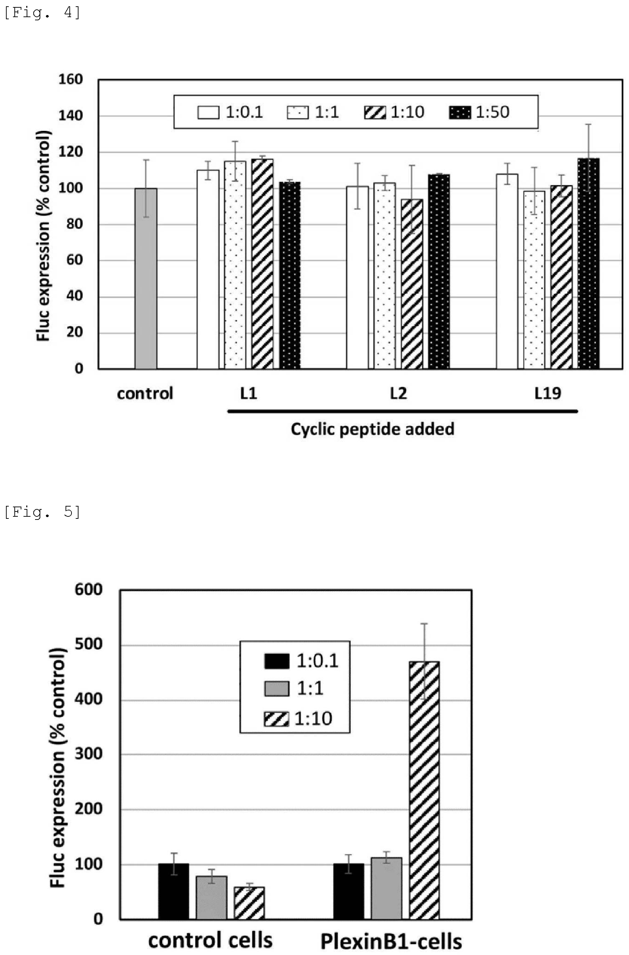

   Fig. 4 shows the transduction activity of HEK293T cells by AAV9 in the presence of three types of cyclic peptides (L1, L2, L19). The amount of peptide added is expressed as an equivalent amount (molar ratio) relative to the AAV9 capsid subunit. Cells were infected with the virus at MOI=100,000, and the activity of the reporter gene luciferase was measured two days later, with the value without peptide added as 100%. Note that, throughout the present specification, the name of the amino acid sequence of the grafted peptide (AAV capsid-binding peptide or target receptor-binding peptide) is represented using the "cyclic peptide name" (e.g., L1, L17, L19, etc.) from which the amino acid sequence is derived. Therefore, even if the amino acid sequence of the grafted peptide itself is different, such as when a linker sequence is added, the same name is used when it is derived from a cyclic peptide that shares a common active structure. Here, "active structure" refers to a partial structure in a cyclic peptide that contains the amino acid sequence essential for affording AAV capsid binding or target receptor binding.
[Fig. 5]
   Fig. 5 shows the effect of the type I engager molecule (L1)₃(PB1m6)₁-SA on enhancing the transduction activity of AAV9 in a Plexin B1-dependent manner. The amount of engager added is expressed as an equivalent amount (molar ratio) relative to the AAV9 capsid subunit. Expi293F cells (control cells) or Plexin B1 forced expression cells (Plexin B1-cells) were infected with the virus-engager complex at MOI=100,000, and the activity of the reporter gene luciferase was measured two days later, with the value without engager added as 100%.
[Fig. 6]

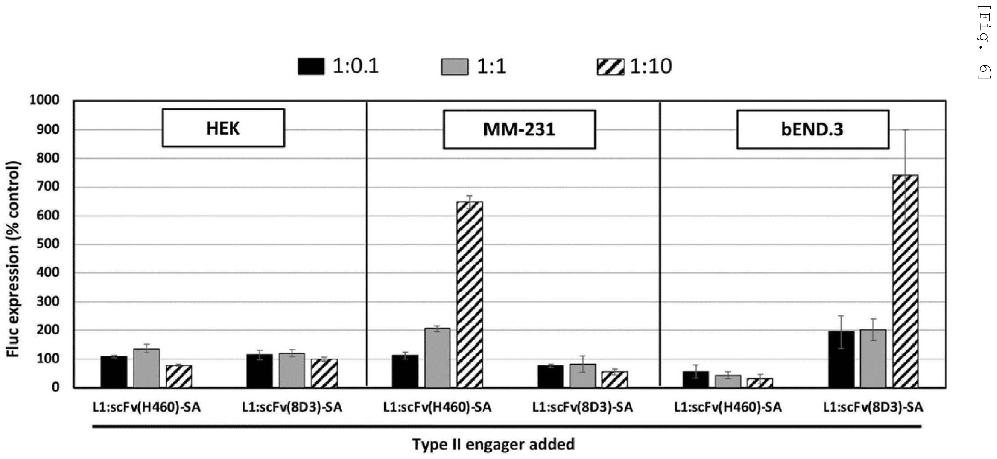

   Fig. 6 shows the effect of the type II engager molecule L1:scFv(H460)-SA and L1:scFv(8D3)-SA on enhancing the transduction activity of AAV9 in an antigen-dependent manner. The amount of engager added is expressed as an equivalent amount (molar ratio) relative to the AAV9 capsid subunit. HEK293T cells, MM-231 cells, or bEND.3 cells were infected with the virus-engager complex at MOI=100,000, and the activity of the reporter gene luciferase was measured two days later, with the value without engager added as 100%.
[Fig. 7]

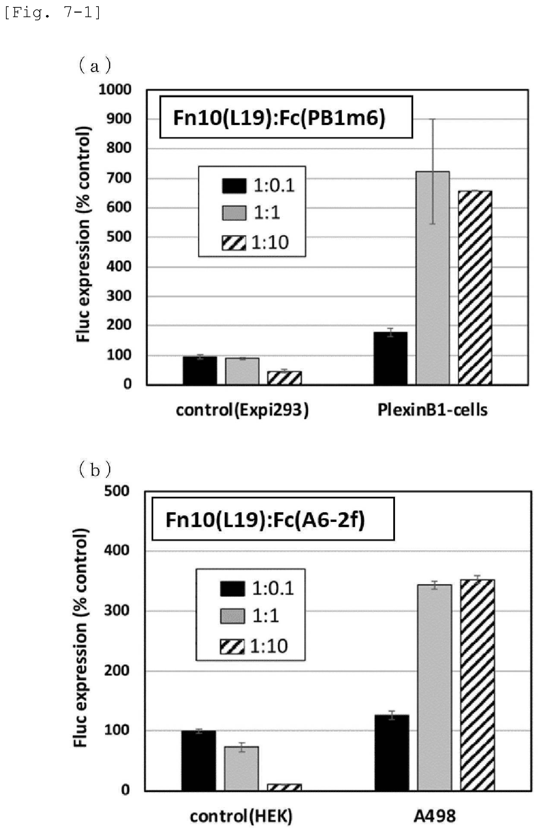

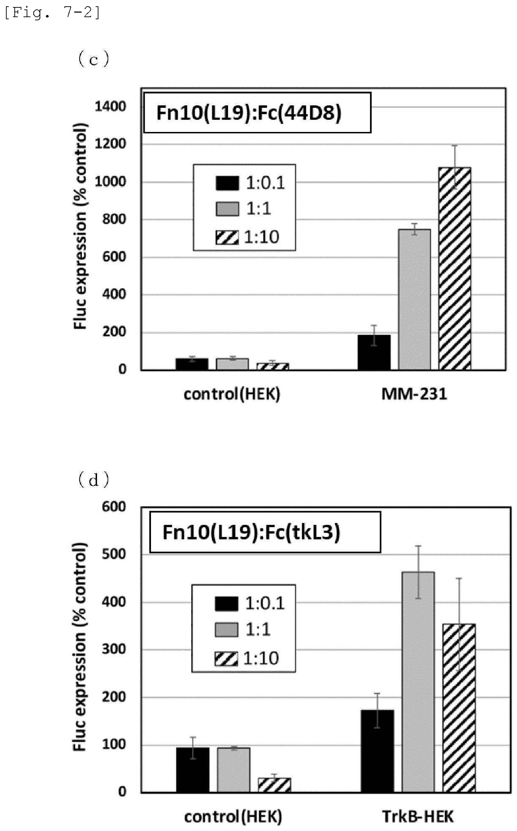

   Fig. 7 shows the effect of various type III engager molecules on enhancing the transduction activity of AAV9 in a target receptor-dependent manner. Fig. 7a shows that Plexin B1-binding engager increases the transduction ability of AAV9 in Plexin B1 forced expression cells, Fig. 7b shows that EGFR-binding engager increases the transduction ability of AAV9 in A498 cells with high EGFR expression, Fig. 7c shows that CD44-binding engager increases the transduction ability of AAV9 in MM-231cells with high CD44 expression, and Fig. 7d shows that TrkB-binding engager increases the transduction ability of AAV9 in TrkB forced expression cells, each in a concentration-dependent and receptor-specific manner. The amount of type III engager added is expressed as an equivalent amount (molar ratio) relative to the AAV9 capsid subunit. Each cell was infected with the virus-engager complex at MOI=100,000, and the activity of the reporter gene luciferase was measured two days later, with the value without engager added as 100%.
[Fig. 8]
   Fig. 8 shows the effect of type III engager molecule grafted with class 1 peptide L17 on enhancing the transduction activity of AAV9 in a target receptor-dependent manner. Fig. 8a shows that Ly6c1-binding engager increases the transduction ability of AAV9 in mouse brain vascular endothelial cell line bEND.3, and Fig. 8b shows that EGFR-binding engager increases the transduction ability of AAV9 in A498 cells with high EGFR expression, each in a concentration-dependent and receptor-specific manner. The amount of type III engager added is expressed as an equivalent amount (molar ratio) relative to the AAV9 capsid subunit. Each cell was infected with the virus-engager complex at MOI=100,000, and the activity of the reporter gene luciferase was measured two days later, with the value without engager added as 100%.
[Fig. 9]
   Fig. 9 shows the effect of type IV engager molecules HSA(L19)₂(44D8)₁ and HSA(L19)₂(L6L41)₁ on enhancing the transduction activity of AAV9 in an antigen-dependent manner. The amount of engager added is expressed as an equivalent amount (molar ratio) relative to the AAV9 capsid subunit. HEK293T cells, MM-231 cells, or bEND.3 cells were infected with the virus-engager complex at MOI=100,000, and the activity of the reporter gene luciferase was measured two days later, with the value without engager added as 100%.
[Fig. 10]

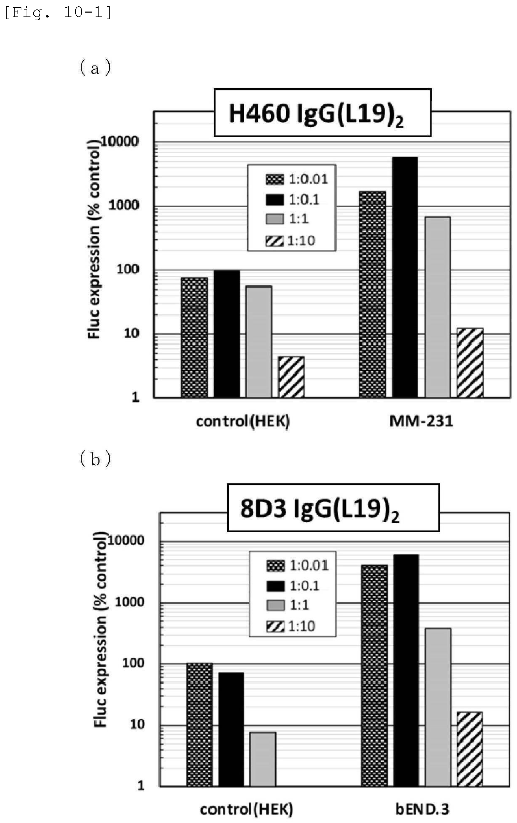

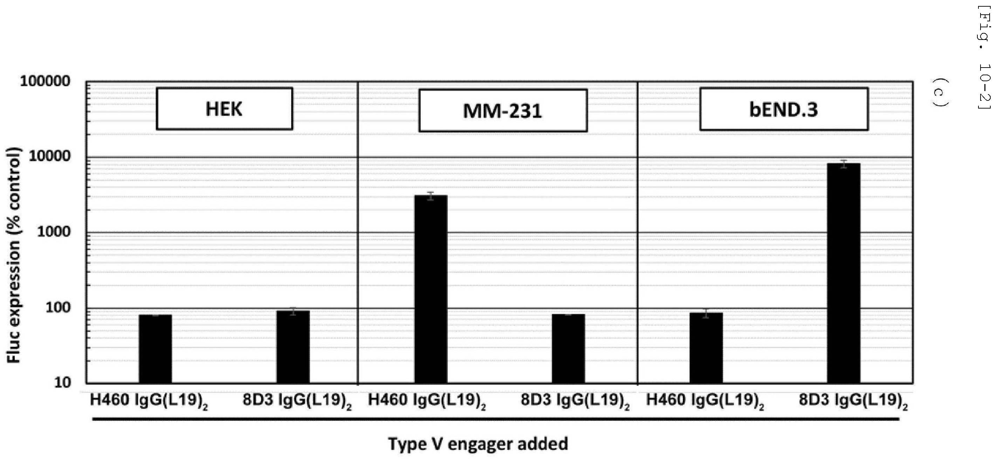

   Fig. 10 shows the effect of type V engager molecules H460-IgG(L19)₂ and 8D3-IgG(L19)₂ on enhancing the transduction activity of AAV9 in an antigen-dependent manner. In Fig. 10a and Fig. 10b, the amount of engager added was varied in molar ratio (N=1) to 0.01, 0.1, 1, and 10 equivalents relative to the AAV9 capsid subunit, HEK293T cells, MM-231 cells, or bEND.3 cells were infected at MOI=100,000, and the activity of the reporter gene luciferase was measured two days later. The relative value to the value without engager added was expressed as a percentage and plotted on a logarithmic scale. In Fig. 10c, the amount of engager added was fixed at 0.1 equivalent, and a similar experiment was performed with N=2 and graphed.
[Fig. 11]

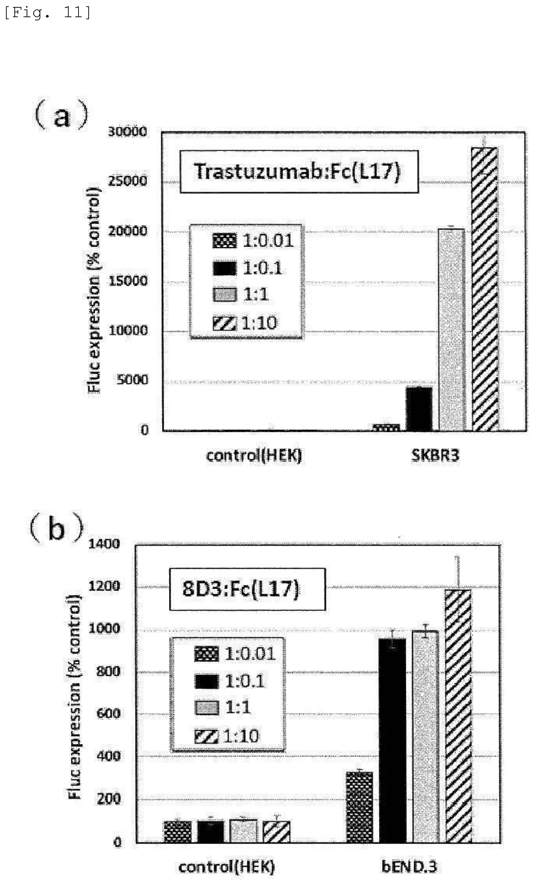

   Fig. 11 shows the effect of type VI engager molecules Trastuzumab:Fn10(L17) and 8D3:Fn10(L17) on enhancing the transduction activity of AAV9 in an antigen-dependent manner. In Fig. 11a and Fig. 11b, the amount of engager added was varied in molar ratio (N=3) to 0.01, 0.1, 1, and 10 equivalents relative to the AAV9 capsid subunit, HEK293T cells, SKBR3 cells, or bEND.3 cells were infected at MOI=100,000, and the activity of the reporter gene luciferase was measured two days later. The relative value to the value without engager added was expressed as a percentage.
[Fig. 12]

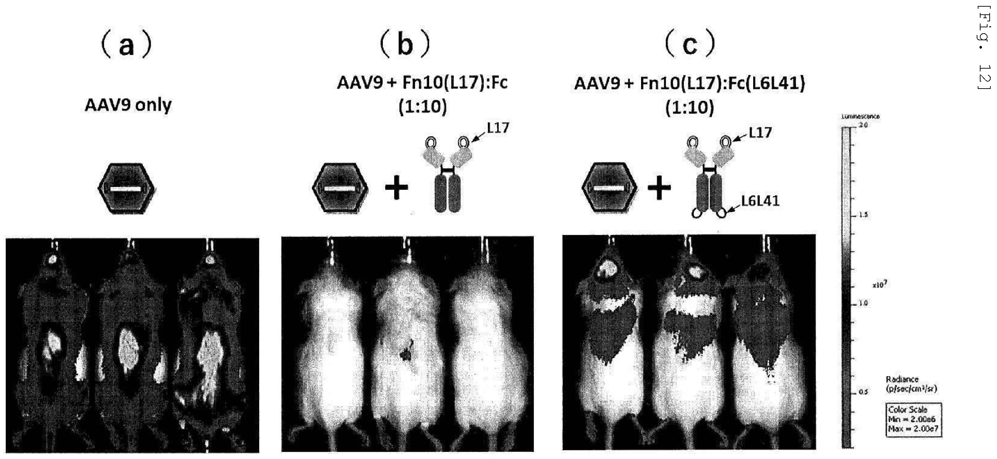

   Fig. 12 shows that AAV9 can be conferred with target receptor specificity in vivo by complexing with an engager. AAV9 or a complex of AAV9 and an engager was administered to the tail vein of ICR mice at 1.5x10¹⁰ vg/mouse, and bioluminescence was measured from the dorsal side 7 days later. The intensity of bioluminescence is indicated by the scale bar on the right end.

### [Description of Embodiments]

The present invention is described in the following by referring to specific embodiments as examples. Unless otherwise specified, all technical terms and scientific terms used in the present specification have the same meaning as commonly understood by one of ordinary skill in the art to which the present invention belongs. In the present specification, unless otherwise specified, amino acid means an L form.

### [Bivalent molecule capable of binding to both AAV capsid and molecular target]

One of the embodiments is directed to
"a molecule comprising a ("AAV capsid-binding ") module (A) capable of binding to a capsid of AAV (adeno-associated virus) and a ("target receptor-binding " module (B) capable of binding to a target receptor, and capable of simultaneously binding to the capsid of AAV and the target receptor, wherein the module (A) is 1) an AAV capsid-binding peptide, or 2) a scaffold protein having a structure in which an AAV capsid-binding peptide is grafted (more specifically, a structure in which an AAV capsid-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein)".

The above-mentioned molecule is a bivalent molecule capable of simultaneously binding to the native AAV capsid and a target receptor. It is added to AAV containing the native AAV capsid to form a complex that acts on cells as a viral vector.

The above-mentioned molecule contains two functional units (modules) which are linked together to form an integrated molecule. The embodiment of linking includes various linking modes, and details are described below. One module binds to the native (unmodified) AAV capsid (also referred to as module (A)), and the other module binds to any target molecule to achieve retargeting (also referred to as module (B)). By binding to the native (unmodified) AAV capsid via module (A) and to cells expressing the target receptor via module (B), the molecule acts as an engager, achieving proximity between AAV and target cells, and enhances the inherent capabilities of AAV to perform attachment, internalization, nuclear translocation, and transduction in a target receptor-dependent manner.

The serotype of AAV to which the molecule is applied is not particularly limited, and can be applied to all known serotypes of AAV. For example, applying to AAV serotypes 1, 2, 5, 6, 8, 9, Rh10, etc. is preferred, and applying to AAV serotype 9 is particularly preferred.

### (Conditions for module (A))

The above-mentioned module (A) is
1) an AAV capsid-binding peptide, or
2) a scaffold protein having a structure in which an AAV capsid-binding peptide is grafted (more specifically, a structure in which an AAV capsid-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein).

Module (A) is not particularly limited as long as it contains an AAV capsid-binding peptide within its molecule that reversibly binds to a native AAV capsid. For example, a preferred embodiment includes a cyclic peptide and one having a partial structure derived from such cyclic peptide, where the cyclic peptide itself does not reduce the inherent transduction activity of AAV (the ability to attach to cells, be internalized, transported to the nucleus, and transduced).

In the present specification, the "AAV capsid-binding peptide" is not particularly limited as long as it has the property of reversibly binding to a native AAV capsid, and may be, for example, all or part of a cyclic peptide that has this property.

The cyclic peptide may be one selected using the RaPID method, and the detail is described below.

In the present specification, the "structure into which an AAV capsid-binding peptide is grafted" more specifically refers to a structure in which all or part of the "AAV capsid-binding peptide" is crosslinked onto a protein by a covalent bond, a structure in which it is tethered to a protein by a non-covalent bond, or a structure in which it is incorporated into the internal sequence of a protein.

Incorporation into the internal sequence of a protein may be achieved by the LassoGraft method, as described in detail below.

In the present specification, the "scaffold protein" is not particularly limited, and examples include immunoglobulin, fibronectin, albumin, ubiquitin, human growth hormone, alkaline phosphatase, retinol-binding protein, uteroglobin, fibrinogen, blood coagulation factors, transferrin, and the like.

Scaffold proteins may be partial fragment proteins, such as partial fragment proteins of the proteins listed above.

The scaffold protein preferably has a loop structure in its secondary structure, and a partial fragment containing a loop structure may be used as a partial structure of the full-length protein.

The loop structure is not particularly limited, and refers to a polypeptide chain region connecting α-helix and/or β-sheet and having a bent structure.

The scaffold protein may also be a protein having a loop structure or a partial fragment protein having a loop structure fused to another protein.

When the AAV capsid-binding peptide constituting the active structure of module (A) is a cyclic peptide, it is desirable that it does not compete with the binding of attachment receptors or internalization receptors when binding to the AAV capsid surface, and that the binding site is located in a recessed region on the capsid surface rather than a protruding portion.

### (Conditions for module (B))

Module (B) is not particularly limited as long as it contains an active structure that reversibly binds to any target receptor on a cell. The target receptor to which this module binds is not particularly limited as long as it is a receptor that exhibits tissue-, organ-, or cell-specific distribution that contributes to AAV retargeting.

The chemical form of module (B) is not particularly limited, and may be any of low-molecular-weight organic compound, linear peptide, cyclic peptide, nucleic acid (including artificial nucleic acid), sugar chain, and protein domain. The cyclic peptide may be one selected using the RaPID method, and the detail is described below.

When the chemical form of module (B) is a protein domain, it may be a protein that originally has the activity to bind to the target molecule (targeted receptor), or it may be a protein that has been artificially engineered to acquire the activity to bind to the target molecule through engineering modification or engineering selection. In the latter case, it may be an artificial binder obtained by inserting a cyclic peptide selected using the RaPID method into any protein domain using the LassoGraft method. The RaPID method and LassoGraft method are described below.

Preferred embodiments of the above-mentioned module (B) include
1) an anti-target receptor antibody,
2) a target receptor-binding peptide,
3) a scaffold protein having a structure in which a target receptor-binding peptide is grafted (more specifically, a structure in which a target receptor-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein), and the like.

In the present specification, the "anti-target receptor antibody" is not particularly limited as long as it is an antibody against a receptor that exhibits tissue-, organ-, or cell-specific distribution that contributes to AAV retargeting. In addition, antibody forms include not only natural IgG types, but also various engineered antibody-like substances such as Fab fragments, single-chain antibodies (scFv), nanobodies, and the like.

In the present specification, "target receptor-binding peptide" is not particularly limited as long as it has the property of reversibly binding to a target receptor protein, and may be, for example, all or part of a cyclic peptide that has this property.

The cyclic peptide may be one selected using the RaPID method, and the detail is described below.

In the present specification, the "structure into which a target receptor-binding peptide is grafted" more specifically refers to a structure in which all or part of the "target receptor-binding peptide" is crosslinked onto a protein by a covalent bond, a structure in which it is tethered to a protein by a non-covalent bond, or a structure in which it is incorporated into the internal sequence of a protein.

Incorporation into the internal sequence of a protein may be achieved by the LassoGraft method, as described in detail below.

### (Embodiment of linking module (A) and module (B))

In the "molecule" described in the present specification, the structure linking module (A) and module (B) may be a peptide bond, glycosidic bond, disulfide bond, or other covalent bond, or may be a non-covalent bond with sufficient chemical stability (e.g., base pair bond, avidin-biotin bond, etc.).

Module (A) and module (B) constituting the "molecule" described in the present specification may be two structurally independent units that can exist independently and stably, or a single structurally inseparable unit that contains both an AAV-binding active site and a target receptor-binding site. In the latter case, although it appears to be a single structure, it functionally contains two modules.

The "molecule" described in the present specification may contain one or more of each of module (A) and module (B). Furthermore, the "molecule" described in the present specification may also be linked to, in addition to module (A) and module (B), one or more structural units having functions unrelated thereto.

In the above, one of the embodiments of the present invention,
"a molecule comprising a ("AAV capsid-binding ") module (A) capable of binding to a capsid of AAV (adeno-associated virus) and a ("target receptor-binding " module (B) capable of binding to a target receptor, and capable of simultaneously binding to the capsid of AAV and the target receptor, wherein module (A) is 1) an AAV capsid-binding peptide, or 2) a scaffold protein having a structure in which an AAV capsid-binding peptide is grafted (more specifically, a structure in which an AAV capsid-binding peptide is crosslinked onto a protein by a covalent bond, a structure in which the peptide is tethered onto a protein by a non-covalent bond, or a structure in which the peptide is incorporated into an internal sequence of a protein, preferably, a structure in which the peptide is incorporated into an internal sequence of a protein)" has been described in detail.

Specific examples of a more preferred embodiment of the molecule include the following.

### Embodiment A

A molecule that is an anti-target receptor antibody having a structure in which an AAV capsid-binding peptide is grafted (type V engager).

### Embodiment B

A molecule that is a protein in which a scaffold protein having a structure in which an AAV capsid-binding peptide is grafted, and a scaffold protein having a structure in which a target receptor-binding peptide is grafted are linked (type III engager).

### Embodiment C

A molecule that is a scaffold protein having a structure in which an AAV capsid-binding peptide and a target receptor-binding peptide are grafted (type IV engager).

### Embodiment D

A molecule that is a protein in which a biotinylated AAV capsid-binding peptide and a biotinylated target receptor-binding peptide are linked via a protein having two or more biotin-binding sites (type I engager).

### Embodiment E

A molecule that is a protein in which a biotinylated AAV capsid-binding peptide and a biotinylated target receptor antibody are linked via a protein having two or more biotin-binding sites (type II engager).

### Embodiment F

A molecule that is a protein in which a scaffold protein having a structure in which an AAV capsid-binding peptide is grafted and an anti-target receptor antibody are linked (type VI engager).

Specific examples of a further preferred embodiment of the molecule include the following.

### Embodiment A-1

A molecule that is an anti-target receptor antibody having an amino acid sequence incorporating all or part of an AAV capsid-binding peptide sequence, which antibody is composed of an anti-target receptor antibody having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.

In the present specification, that "all or part of the amino acid sequence constituting the loop structure is replaced with all or part of an AAV capsid-binding peptide sequence" means that all or part of the amino acid sequence constituting the loop structure is deleted, and in place of that sequence, all or part of an AAV capsid-binding peptide sequence is incorporated as part of the amino acid sequence constituting the loop structure.

In the present specification, that "all or part of an AAV capsid-binding peptide sequence is inserted into the amino acid sequence constituting the loop structure" means that all or part of the AAV capsid-binding peptide sequence is inserted between two adjacent amino acids in the amino acid sequence constituting the loop structure, and incorporated as part of the amino acid sequence constituting the loop structure.

### Embodiment B-1

A molecule that is a protein to which
an AAV capsid-binding scaffold protein having an amino acid sequence incorporating all or part of an AAV capsid-binding peptide sequence, which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and
a target receptor-binding scaffold protein having an amino acid sequence incorporating all or part of a target receptor-binding peptide sequence, which target receptor-binding scaffold protein is composed of a scaffold protein having at least one loop structure wherein all or part of an amino acid sequence constituting the at least one loop structure is replaced with all or part of the target receptor-binding peptide sequence, or all or part of the target receptor-binding peptide sequence is inserted into an amino acid sequence constituting the at least one loop structure
are linked.

### Embodiment C-1

A molecule that is a protein having an amino acid sequence incorporating all or part of each of an AAV capsid-binding peptide sequence and a target receptor-binding peptide sequence, which protein is composed of a scaffold protein having two or more loop structures wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and wherein all or part of an amino acid sequence constituting at least one loop structure different from the above-mentioned loop structure is replaced with all or part of the target receptor-binding peptide sequence, or all or part of the target receptor-binding peptide sequence is inserted into an amino acid sequence constituting the at least one loop structure different from the above-mentioned loop structure.

### Embodiment D-1

A molecule that is a protein in which a biotinylated AAV capsid-binding peptide and a biotinylated target receptor-binding peptide are linked via avidin or streptavidin.

### Embodiment E-1

A molecule that is a protein in which a biotinylated AAV capsid-binding peptide and a biotinylated target receptor antibody are linked via avidin or streptavidin.

### Embodiment F-1

A protein to which
an anti-target receptor antibody and an AAV capsid-binding scaffold protein having an amino acid sequence incorporating all or part of an AAV capsid-binding peptide sequence are linked,
which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.

### [Cyclic peptide]

In the above-mentioned [bivalent molecule capable of binding to both AAV capsid and molecular target], which is one of the embodiments of the present invention, the AAV capsid-binding peptide and target receptor-binding peptide may be the entire sequence or a partial sequence of the AAV capsid-binding cyclic peptide sequence and the target receptor-binding cyclic peptide sequence.

The AAV capsid-binding cyclic peptide and the target receptor-binding cyclic peptide are described in detail below. This cyclic peptide, as a cyclic peptide itself, constitutes one of the embodiments of the present invention.

### (AAV capsid-binding cyclic peptide)

In the present specification, one embodiment of the AAV capsid-binding cyclic peptide is a "cyclic peptide comprising an AAV capsid-binding amino acid sequence in the peptide sequence" (Embodiment (I)).

As used herein, the AAV capsid-binding amino acid sequence is not particularly limited as long as it is an amino acid sequence that reversibly binds to a native AAV capsid.

As described above, this cyclic peptide is useful, for example, for constructing bivalent molecules that can bind to both an AAV capsid and a molecular target.

More preferred specific embodiments of the above-mentioned embodiment (I) include the following embodiments (1) to (12).

### Embodiment (1)

The cyclic peptide described in the above-mentioned (I), having a cyclic structure formed via a crosslinking bond connecting one amino acid to another amino acid in the peptide sequence.

### Embodiment (2)

The cyclic peptide described in the above-mentioned (I), wherein the AAV capsid-binding cyclic peptide has a cyclic structure containing 4 to 30 amino acids.

### Embodiment (3)

The cyclic peptide described in the above-mentioned (1) or (2), wherein the crosslinking structure contains a thioether bond or a disulfide bond.

### Embodiment (4)

The cyclic peptide described in any of the above-mentioned (1) to (3), wherein the AAV capsid-binding cyclic peptide has a cyclic structure formed via a crosslinking bond connecting an amino acid having a Functional Group 1 ("ring-forming amino acid 1") shown in Functional Group groups (A) to (E) in the following Table 10, with an amino acid having the corresponding Functional Group 2 ("ring-forming amino acid 2").

**[Table 10]**

| | Functional Group 1 | Functional Group 2 |
|---|---|---|
| (A) | 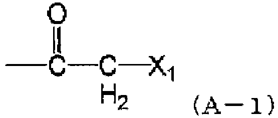 | HS- (A-2) |
| (B) | -C≡C-H (B-1) | N₃- (B-2) |
| (C) | -Ar-CH₂NH₂ (C-1) | 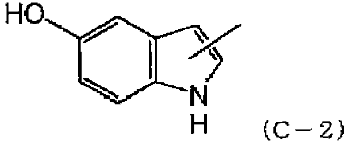 |
| (D) | -C≡C-CH₂-X₁ (D-1) | HS- (D-2) |
| (E) | -Ar-CH₂-X₁ (E-1) | HS- (E-2) |

| | | |
|---|---|---|
| wherein X₁ is a leaving group and Ar is an aromatic ring optionally having substituent(s). | | |

### Embodiment (5)

The cyclic peptide described in the above-mentioned (4), wherein the ring-forming amino acid 1 is an amino acid having a Functional Group (A-1) described in the aforementioned Table 1, the ring-forming amino acid 2 is an amino acid having the corresponding Functional Group (A-2), and the cyclic structure comprises an -N-CO-CH₂-S- structure formed between the both amino acids.

### Embodiment (6)

The cyclic peptide described in the above-mentioned (4), wherein N in the -N-CO-CH₂-S- structure is derived from an amino group of the ring-forming amino acid 1 selected from alanine, tyrosine, phenylalanine, tryptophan, ornithine, lysine, diaminopropionic acid, and diaminobutyric acid.

### Embodiment (7)

The cyclic peptide described in the above-mentioned (4), wherein S in the -N-CO-CH₂-S- structure is derived from a thiol group of the ring-forming amino acid 2 selected from cysteine, homo cysteine, mercaptonorvaline, mercaptonorleucine, 2-amino-7-mercaptoheptanoic acid, and 2-amino-8-mercaptooctanoic acid.

### Embodiment (8)

The cyclic peptide described in any of the above-mentioned (1) to (7), wherein the AAV capsid-binding cyclic peptide is a cyclic peptide containing, in the peptide sequence, an ("AAV9 capsid-binding ") amino acid sequence capable of binding to a capsid of AAV9 (adeno-associated virus serotype 9) ("AAV9 capsid-binding cyclic peptide").

### Embodiment (9)

The cyclic peptide described in the above-mentioned (8), wherein the AAV9 capsid-binding cyclic peptide has a cyclic structure formed via
1) a crosslinking bond connecting one amino acid located at one end of the AAV9 capsid-binding amino acid sequence or located outside that end to another amino acid located at the other end of the AAV9 capsid-binding amino acid sequence or located outside that end, or
2) a crosslinking bond connecting one amino acid located at one end of the AAV9 capsid-binding amino acid sequence or located outside that end to another amino acid located within the AAV9 capsid-binding amino acid sequence.

### Embodiment (10)

The cyclic peptide described in the above-mentioned (9), wherein the AAV9 capsid-binding amino acid sequence is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn.

### Embodiment (11)

The cyclic peptide described in the above-mentioned (9), wherein the core motif sequence (II) is
(1) the amino acid sequence shown in Tyr-Ser-Lys-Ser-Val-Cys-Leu-Ala-Gln-Asp (SEQ ID NO: 2), or
   an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.

### Embodiment (12)

The cyclic peptide described in any of the above-mentioned (8) to (11), wherein the active structure of the AAV9 capsid-binding cyclic peptide is selected from cyclic peptides represented by the following formula (Ia), the formula (Ib), and the formula (Ic): 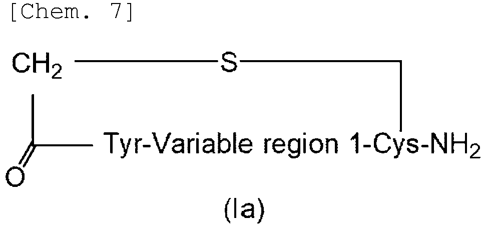
wherein Variable region 1 is an AAV9-binding amino acid sequence selected from the amino acid sequences listed in the following Table 11, and
S is S derived from the thiol group of Cys

**[Table 11]**

| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

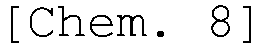

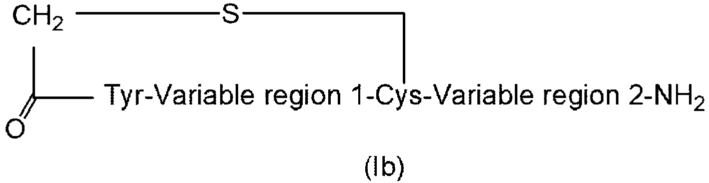

wherein Variable region 1 is an AAV9-binding amino acid sequence selected from the amino acid sequences listed in the following Table 12,
Variable region 2 is an AAV9-binding amino acid sequence selected from the amino acid sequences listed in the following Table 13, and
S is S derived from the thiol group of Cys

**[Table 12]**

| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |

wherein an amino acid sequence of Variable region 1 in Table 12 is preferably Ser Lys Ser Val (SKSV),

**[Table 13]**

| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

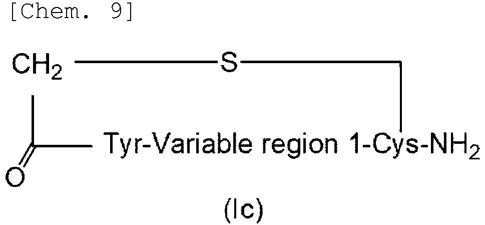

wherein Variable region 1 is an AAV9-binding amino acid sequence selected from the amino acid sequences listed in the following Table 14, and
S is S derived from the thiol group of Cys

**[Table 14]**

| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 . | Arg Phe Ser Lys Ser Leu His Leu Ala Gln Gln Tyr Gln Leu Ile (RFSKSLHLAQQYQLI) | 23 |

The above-mentioned AAV capsid-binding cyclic peptide can be used in either a free form or a form of a salt thereof (preferably, a pharmaceutically acceptable salt thereof).

Pharmaceutically acceptable salts include, for example, salts with inorganic acids such as hydrochloride, hydrobromide, sulfate, and phosphate, salts with organic acids such as acetate, fumarate, oxalate, citrate, methanesulfonate, benzenesulfonate, tosylate, and maleate, salts with bases such as alkali metal salts such as sodium salt and potassium salt, and alkaline earth metal salts such as calcium salt, and salts with amino acids such as glycine salt, lysine salt, arginine salt, histidine salt, ornithine salt, glutamate, and aspartate.

In the above, the characteristics of the ring structure and amino acid sequence of the AAV capsid-binding cyclic peptide in the embodiments of the present invention are described in detail. As described above, the AAV capsid-binding peptide in the embodiments of the present invention may be the entire sequence or a partial sequence of the AAV capsid-binding cyclic peptide sequence. From this viewpoint, preferred amino acid sequences of the "AAV capsid-binding peptide" in the embodiments described as specific embodiments [1] to [12] of the AAV retargeting strategy in the above section [Means of Solving the Problems] are described below.

### (Sequence A)

A preferred sequence of the AAV capsid-binding peptide is Sequence A which is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn, or
a sequence comprising the sequence.

### (Sequence B)

A more preferred sequence of the AAV capsid-binding peptide is the above-mentioned Sequence A wherein the core motif sequence (II) is
(1) the amino acid sequence shown in Tyr-Ser-Lys-Ser-Val-Cys-Leu-Ala-Gln-Asp (SEQ ID NO: 2), or
   an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.

### (Sequence C)

Another preferred sequence of the AAV capsid-binding peptide is Sequence C which is a full sequence or a partial sequence constituting the cyclic peptide represented by the following formula (Ia), the formula (Ib), or the formula (Ic). 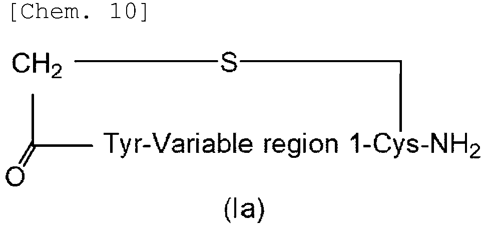
wherein Variable region 1 is an AAV9-binding amino acid sequence selected from the amino acid sequences listed in the following Table 15, and
S is S derived from the thiol group of Cys

**[Table 15]**

| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

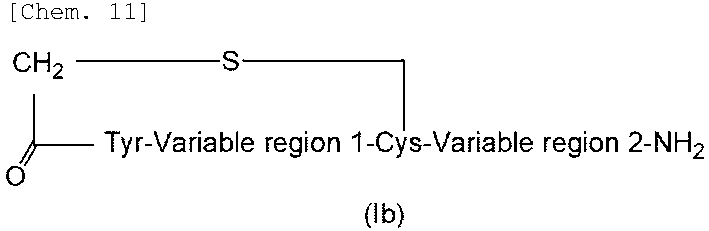

wherein Variable region 1 is an AAV9-binding amino acid sequence selected from the amino acid sequences listed in the following Table 16,
Variable region 2 is an AAV9-binding amino acid sequence selected from the amino acid sequences listed in the following Table 17, and
S is S derived from the thiol group of Cys

**[Table 16]**

| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |

wherein the amino acid sequence of Variable region 1 in Table 16 is preferably Ser Lys Ser Val (SKSV),

**[Table 17]**

| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

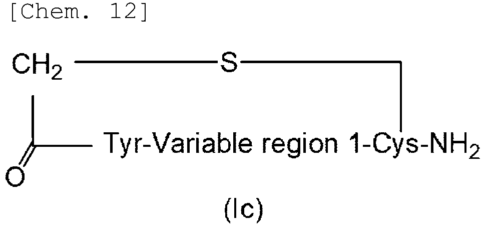

wherein Variable region 1 is an AAV9-binding amino acid sequence selected from the amino acid sequences listed in the following Table 18, and
S is S derived from the thiol group of Cys

**[Table 18]**

| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |

### (Target receptor-binding cyclic peptide)

In the present specification, one embodiment of the target receptor-binding cyclic peptide is a "cyclic peptide comprising a target receptor-binding amino acid sequence in the peptide sequence" (Embodiment (II)).

As used herein, the target receptor-binding amino acid sequence is not particularly limited as long as it is an amino acid sequence that reversibly binds to a target receptor.

As described above, this cyclic peptide is useful, for example, for constructing bivalent molecules that can bind to both an AAV capsid and a molecular target.

More preferred specific embodiments of the above-mentioned embodiment (II) include the following Embodiment (a).

### Embodiment (a)

The cyclic peptide described in the above-mentioned (II), having a cyclic structure formed via a crosslinking bond connecting one amino acid to another amino acid in the peptide sequence.

In the following, embodiments of a more preferred target receptor-binding cyclic peptide include Embodiments (2) to (7) described above as preferred examples of the AAV-binding cyclic peptide.

The above-mentioned target receptor-binding cyclic peptides can also be used in either free form or in the form of a salt thereof (preferably, a pharmaceutically acceptable salt thereof). Pharmaceutically acceptable salts include the salts described above for the AAV capsid-binding cyclic peptides.

### (Production method of cyclic peptide)

The above-described cyclic peptides are cyclic peptides that can be produced using known peptide synthesis techniques. Methods for producing cyclic peptides include, for example, chemical synthesis methods such as the liquid-phase method, the solid-phase method, and a hybrid method combining the liquid-phase and solid-phase methods, recombinant DNA methods, and translational synthesis using a cell-free translation system.

It is preferable to use cyclic peptides that can be suitably produced by mRNA display methods such as the RaPID method and the TRAP method, or phage display methods. When practicing the present invention, production by the RaPID method is particularly preferred.

The RaPID method combines translational synthesis of a specialized cyclic peptide library with mRNA display to enrich for active species that bind to a target protein, and further, the cDNA encoding that active species is recovered and amplified by PCR to allow for transcription of the mRNA again. This process is repeated to identify cyclic peptides having high affinity and specificity (see WO 2011/049157, JP 2013-46637 A, and the like).

The method for cyclizing a peptide is not particularly limited. For example, by including an amino acid having Functional Group 1 and an amino acid having the corresponding Functional Group 2, as shown in Table 19 below, a peptide synthesized through translational synthesis by a spontaneous reaction can be cyclized to produce a cyclic peptide.

Either Functional Group 1 or 2 can be located on the N-terminus, or they can be located at the N-terminus and the C-terminus. Alternatively, one can be a terminal amino acid and the other can be a non-terminal amino acid, or both can be non-terminal amino acids.

The bond formed by Functional Group 1 and Functional Group 2 can be said to be a chemical crosslinking structure that forms the molecular cyclic structure of the cyclic peptide.

**[Table 19]**

| | Functional Group 1 | Functional Group 2 |
|---|---|---|
| (A) | 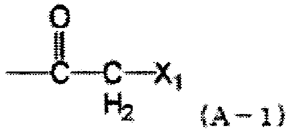 | HS- (A-2) |
| (B) | -C≡C-H (B-1) | N₃- (B-2) |
| (C) | -Ar-CH₂NH₂ (C-1) | 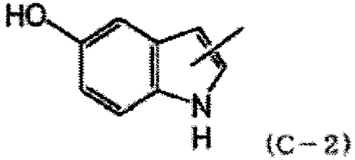 |
| (D) | -C≡C-CH₂-X₁ (D-1) | HS- (D-2) |
| (E) | -Ar-CH₂-X₁ (E-1) | HS- (E-2) |

| | | |
|---|---|---|
| wherein X₁ is a leaving group, such as a halogen atom (e.g., Cl, Br, I, etc.), and Ar is an aromatic ring (e.g., a benzene ring) that may have a substituent. | | |

In the phage display method, Cys can be bonded together to form a cyclic peptide. Therefore, a cyclic peptide having a disulfide bond between -SH as Functional Group 1 and HS- as Functional Group 2 can be formed.

In the RaPID method, for example, a chloroacetylated amino acid can be used as the amino acid having the Functional Group (A-1). Examples of the chloroacetylated amino acid include N-chloroacetyl-L-alanineN-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophane, β-N-chloroacetyl-L-diaminopropanoic acid, γ-N-chloroacetyl-L-diaminobutyric acid, σ-N-chloroacetyl-L-ornithine, ε-N-chloroacetyl-L-lysine, and corresponding D-amino acid derivatives, and the like.

N-chloroacetyl-L-tryptophan and N-chloroacetyl-L-tyrosine are preferably used as amino acids having the Functional Group (A-1).

Examples of the amino acid having the Functional Group (A-2) include cysteine, homocysteine, mercaptonorvaline, mercaptonorleucine, 2-amino-7-mercaptoheptanoic acid, 2-amino-8-mercaptooctanoic acid, and the like.

Cysteine is preferably used as amino acid having the Functional Group (A-1).

Cyclization methods using an amino acid having Functional Group (A-1) and an amino acid having Functional Group (A-2) include, for example, methods described in Kawakami, T. et al., Nature Chemical Biology 5, 888-890 (2009); Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); Kawakami T. et al, Chemistry & Biology 15, 32-42 (2008), WO 2008/117833, and the like.

Examples of the amino acid having the Functional Group (B-1) include propargylglycine, homopropargylglycine, 2-amino-6-heptynoic acid, 2-amino-7-octynoic acid, and 2-amino-8-nonynoic acid, and the like.

4-Pentynoylated or 5-hexynoylated amino acids may also be used.

Examples of the 4-pentynoylated amino acid include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophane, β -N-(4-pentenoyl)-L-diaminopropanoic acid, γ -N-(4-pentenoyl)-L-diaminobutyric acid, σ-N-(4-pentenoyl)-Lornithine, ε-N-(4-pentenoyl)-L-lysine, D-amino acid derivatives corresponding thereto, and the like.

Examples of the 5-hexynoylated amino acid include those in which the 4-pentynoyl group in the compounds exemplified as 4-pentynoylated amino acids has been replaced with a 5-hexynoyl group.

Examples of the amino acid having the Functional Group (B-2) include azidoalanine, 2-amino-4-azidobutanoic acid, azidoptonorvaline, azidonorleucine, 2-amino-7-azidoheptanoic acid, 2-amino-8-azidooctanoic acid, and the like.

Azidoacetylated or 3-azidopentanoylated amino acids can also be used.

Examples of the azidoacetylated amino acid include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophane, β-N-azidoacetyl-L-diaminopropanoicacid, γ-N-azidoacetyl-L-diaminobutyric acid, σ-N-azidoacetyl-L-ornithine, ε-N-azidoacetyl-L-lysine, D-amino acid derivatives corresponding thereto, and the like.

Examples of the 3-azidopentanoylate amino acid include those in which the azidoacetyl group in the compounds exemplified as azidoacetylated amino acids has been replaced with a 3-azidopentanoyl group.

Cyclization methods using an amino acid having Functional Group (B-1) and an amino acid having Functional Group (B-2) include, for example, methods described in Sako, Y. et al., Journal of American Chemical Society 130, 7932-7934 (2008), WO 2008/117833, and the like.

Examples of the amino acid having the Functional Group (C-1) include N-(4-aminomethyl-benzoyl)-phenylalanine (AMBF), 3-aminomethyltyrosine, and the like.

Examples of the amino acid having the Functional Group (C-2) include 5-hydroxytryptophan (WOH) and the like.

Cyclization methods using an amino acid having Functional Group (C-1) and an amino acid having Functional Group (C-2) include, for example, methods described in Yamagishi, Y. et al., Chem Bio Chem 10, 1469-1472 (2009), WO 2008/117833, and the like.

Examples of the amino acid having Functional Group (D-1) include 2-amino-6-chloro-hexynoic acid, 2-amino-7-chloro-heptynoic acid, 2-amino-8-chloro-octynoic acid, and the like.

Examples of the amino acid having Functional Group (D-2) include cysteine, homocysteine, mercaptonorvaline, mercaptonorleucine, 2-amino-7-mercaptoheptanoic acid, 2-amino-8-mercaptooctanoic acid, and the like.

Cyclization methods using an amino acid having Functional Group (D-1) and an amino acid having Functional Group (D-2) include, for example, methods described in WO 2012/074129 and the like.

Examples of the amino acid having Functional Group (E-1) include N-3-chloromethylbenzoyl-L-phenylalanine, N-3-chloromethylbenzoyl-L-tyrosine, N-3-chloromethylbenzoyl-L-tryptophane, D-amino acid derivatives corresponding thereto, and the like.

Examples of the amino acid having Functional Group (E-2) include cysteine, homocysteine, mercaptonorvaline, mercaptonorleucine, 2-amino-7-mercaptoheptanoic acid, 2-amino-8-mercaptooctanoic acid, and the like.

Cyclization methods using an amino acid having Functional Group (E-1) and an amino acid having Functional Group (E-2) can be performed with reference to, for example, the cyclization methods using (A-1) and (A-2) or the cyclization methods using (D-1) and (D-2).

As the ring-forming amino acid, a combination of an amino acid having Functional Group (A-1) and an amino acid having Functional Group (A-2) is preferred, a combination of N-acetyltryptophan in which H is substituted with a leaving group and cysteine is more preferred, and a combination of N-haloacetyl-D-tyrosine or N-haloacetyl-D-tryptophan, preferably N-chloroacetyl-D-tyrosine or N-chloroacetyl-D-tryptophan, and cysteine (Cys) is further preferred.

The cyclic peptides used in the present invention can be produced according to the RaPID method described above. Details of the production of the cyclic peptide described in the aforementioned Embodiment (12) are described in the [Examples] section.

As described in embodiments [6] to [8] in the aforementioned [Means of Solving the Problems] section, one embodiment of the present invention is an embodiment in which all or part of a cyclic peptide sequence is incorporated into the amino acid sequence of a scaffold protein having at least one loop structure and the like. Such incorporation of a cyclic peptide sequence can be performed using standard genetic engineering techniques, and the LassoGraft method is preferably used. The LassoGraft method is described in detail in Japanese Patent No. 6598344, and those skilled in the art can practice this method with reference to this document. The key points are described below.

Taking the above-mentioned Embodiments [6] to [8] as an example, all or part of a cyclic peptide sequence is incorporated into the loop structure of an anti-target receptor antibody or scaffold protein (hereinafter also abbreviated as "target protein") having at least one loop structure, and this "incorporation" is preferably via a covalent bond. Here, "incorporation" refers to replacing all or part of the amino acid sequence constituting the above-mentioned loop structure with all or part of a cyclic peptide sequence, or inserting all or part of a cyclic peptide sequence into the above-mentioned amino acid sequence constituting the loop structure.

The site into which the cyclic peptide is incorporated in the loop structure is not particularly limited, and all or part of the cyclic peptide sequence (hereinafter also abbreviated as "cyclic peptide sequence") may be inserted at any site in the loop structure.

"Any site in the loop structure" means that two amino acid residues for incorporating the cyclic peptide are selected from within the loop structure.

Preferably, the two amino acid residues in the loop structure are preferably located such that the distance between the Cα atoms is 4 to 7 Å, and both are at least partially exposed on the protein surface. With the distance between the Cα atoms of the two amino acid residues of 4 to 7 Å, the cyclic peptide incorporated into the target protein more easily maintains the structure of the original cyclic peptide also in the modified peptide.

The distance between the Cα atoms of two amino acid residues can be confirmed from structural data of a target protein having a known three-dimensional structure. Even when the three-dimensional structure of the target protein is unknown, the distance between the two amino acid residues can be estimated if a model structure constructed from structural information of a similar protein (homologue) is available.

The two amino acid residues selected for incorporation of a cyclic peptide within a loop structure may be adjacent amino acid residues present in the loop structure, and it is preferable that the two amino acid residues are not adjacent. "Two amino acid residues not adjacent" is synonymous with the two amino acid residues being discontinuous in the primary amino acid sequence in the protein loop structure.

For example, when selecting two amino acid residues whose arrangement in the protein crystal structure is such that the distance between the Cα atoms of the two amino acid residues is 4 to 7 Å, there may be 1 to 15 amino acid residues between the two amino acid residues constituting the loop structure in the target protein. That 1 to 15 amino acid residues are present between two amino acid residues is synonymous with that the two amino acid residues are separated by 1 to 15 amino acid residues in the primary amino acid sequence of a loop structure of a protein.

The 1 to 15 amino acid residues between the two amino acid residues are preferably amino acid residues that form a loop structure.

In a method for presenting a cyclic peptide on a protein having a loop structure, the cyclic peptide is presented on the protein having a loop structure, and preferably, the loop structure is replaced with the cyclic peptide, thereby maintaining the structure of the protein and the structure of the cyclic peptide. By selecting two amino acid residues from the loop structure to be bound to the cyclic peptide such that the distance between the Cα atoms of the two amino acid residues is 4 to 7 Å, the original structure of the protein can be maintained while the activity of the cyclic peptide can be preserved.

In an embodiment of the present invention, when "replacing the chemical crosslinking structure of a cyclic peptide with two amino acid residues constituting a loop structure", the chemical crosslinking structure of the cyclic peptide may be replaced with the two amino acid residues constituting the loop structure via a linker sequence. When replacing via a linker sequence, in a modified protein into which a cyclic peptide has been incorporated, the amino acid sequence derived from the loop structure, the linker sequence, and the amino acid sequence derived from the cyclic peptide are incorporated in this order at the N-terminus, and the amino acid sequence derived from the cyclic peptide, the linker sequence, and the amino acid sequence derived from the loop structure are incorporated in this order at the C-terminus. Linker sequences may be present at both the N-terminus and the C-terminus, or at either the N-terminus or the C-terminus. Linker sequences include, but are not limited to, sequences consisting of one or more of Ser, Gly, and Cys.

In an embodiment of the present invention, a cyclic peptide is incorporated into a target protein having a loop structure, and the incorporation can be performed using conventional genetic engineering techniques.

Specifically, a modified protein incorporating a cyclic peptide can be produced by the following procedure.
1) From the amino acid sequence of a cyclic peptide having a chemical crosslinking structure for forming an intramolecular cyclic structure, an amino acid sequence to be incorporated into a target protein is selected, and a base sequence corresponding to the amino acid sequence is selected. Where necessary, adjustments are made to incorporate a linker sequence.
2) Base sequences corresponding to two amino acid residues that constitute the loop structure of a target protein having a loop structure are selected, bases present between the selected base sequences are deleted as necessary, and a base sequence corresponding to the amino acid sequence of the selected cyclic peptide (including a linker sequence as necessary) is inserted to prepare a nucleic acid having the incorporated base sequence.
3) The nucleic acid is translated to obtain a target protein incorporating a cyclic peptide.

The method for selecting base sequences corresponding to two amino acid residues that constitute the loop structure of a target protein having a loop structure and deleting bases present between the selected base sequences as necessary, and the method for preparing a nucleic acid having a base sequence constructed by inserting a base sequence corresponding to the amino acid sequence of the selected cyclic peptide are not particularly limited, and can be performed using conventionally known methods. In addition, methods for translating prepared nucleic acids are already well known in the technical field to which the present invention pertains, and therefore, nucleic acids may be translated by appropriately applying these well-known methods.

Various embodiments of "the molecule" described in the present specification are described in detail above. More specific embodiments are recited below.
(1) In some embodiments, module (A) is a protein that presents an AAV-binding cyclic peptide, and the protein may be a fibronectin type III module (FN3) fragment, an Fc region fragment of an antibody, streptavidin, serum albumin, ubiquitin, or the like. The method for grafting the cyclic peptide onto these proteins may be a ligation reaction using a chemical crosslinking agent, a crosslinking reaction using Cys residue, a non-covalent ligation reaction using biotin-avidin interaction, or a grafting method including genetically engineering a recombinant protein incorporating the internal sequence of an AAV-binding cyclic peptide into the loop using the LassoGraft method.
(2) In some embodiments, module (A) may be an artificial protein that has acquired AAV-binding ability by incorporating, by the LassoGraft method, an internal sequence of an AAV-binding cyclic peptide into one or two of the superficial loops of an FN3 fragment.
(3) In some embodiments, module (A) may be an artificial protein that has acquired AAV-binding ability by incorporating, by the LassoGraft method, an internal sequence of an AAV-binding cyclic peptide into one or two of the superficial loops of an Fc fragment of an antibody.
(4) In some embodiments, module (A) may be an artificial protein that has acquired AAV-binding ability by incorporating, by the LassoGraft method, an internal sequence of an AAV-binding cyclic peptide into one or two of the superficial loops of serum albumin.
(5) In some embodiments, module (A) may be an artificial protein that has acquired AAV-binding ability by incorporating, by the LassoGraft method, an internal sequence of an AAV-binding cyclic peptide into one of the superficial loops of ubiquitin, or an artificial protein formed by linking a plurality of such proteins.
(6) In some embodiments, module (B) may be an artificial protein that has acquired target receptor-binding ability by incorporating, by the LassoGraft method, an internal sequence of a target receptor-binding cyclic peptide into one or two of the superficial loops of an FN3 fragment.
(7) In some embodiments, module (B) may be an artificial protein that has acquired target receptor-binding ability by incorporating, by the LassoGraft method, an internal sequence of a target receptor-binding cyclic peptide into one or two of the superficial loops of an Fc fragment of an antibody.
(8) In some embodiments, module (B) may be an artificial protein that has acquired target receptor-binding ability by incorporating, by the LassoGraft method, an internal sequence of a target receptor-binding cyclic peptide into one or two of the superficial loops of serum albumin.
(9) In some embodiments, module (B) may be an artificial protein that has acquired target receptor-binding ability by incorporating, by the LassoGraft method, an internal sequence of a target receptor-binding cyclic peptide into one of the superficial loops of ubiquitin, or an artificial protein formed by linking a plurality of such proteins.
(10) In some embodiments, module (B) may be a full-length protein, Fab fragment, or scFv fragment of an IgG-type anti-target receptor antibody.
(11) In some embodiments, module (B) may be an anti-target receptor nanobody.
(12) In some embodiments, the "molecule" described in the present specification may be a homodimeric protein obtained by fusing, via the hinge sequence of an antibody, module (A) that has acquired AAV-binding ability by incorporating, by the LassoGraft method, the internal sequence of an AAV-binding cyclic peptide into one or two of the superficial loops of an FN3 fragment, with module (B) that has acquired target receptor-binding ability by incorporating, by the LassoGraft method, the internal sequence of a target receptor-binding cyclic peptide into one or two of the superficial loops of an Fc fragment of an antibody.
(13) In some embodiments, the "molecule" described in the present specification may be a homodimeric protein obtained by fusing, via the hinge sequence of an antibody, module (A) that has acquired AAV-binding ability by incorporating, by the LassoGraft method, the internal sequence of an AAV-binding cyclic peptide into one or two of the superficial loops of an Fc fragment of an antibody, with module (B) that has acquired target receptor-binding ability by incorporating, by the LassoGraft method, the internal sequence of a target receptor-binding cyclic peptide into one or two of the superficial loops of an FN3 fragment.
(14) In some embodiments, the "molecule" described in the present specification may be a homodimeric protein obtained by fusing, via the hinge sequence of an antibody, module (A) linking one or more artificial proteins that have acquired AAV-binding ability by incorporating, by the LassoGraft method, the internal sequence of an AAV-binding cyclic peptide into one of the superficial loops of ubiquitin, with module (B) that has acquired target receptor-binding ability by incorporating, by the LassoGraft method, the internal sequence of a target receptor-binding cyclic peptide into one or two of the superficial loops of an Fc fragment of an antibody.
(15) In some embodiments, the "molecule" described in the present specification may be a homodimeric protein obtained by fusing, via the hinge sequence of an antibody, module (A) that has acquired AAV-binding ability by incorporating, by the LassoGraft method, the internal sequence of an AAV-binding cyclic peptide into one or two of the superficial loops of an Fc fragment of an antibody, with module (B) linking one or more artificial proteins that have acquired target receptor-binding ability by incorporating, by the LassoGraft method, the internal sequence of a target receptor-binding cyclic peptide into one of the superficial loops of ubiquitin.
(16) In some embodiments, "the molecule" described in the present specification may be an IgG antibody-like protein that has acquired AAV-binding ability by incorporating, by the LassoGraft method, the internal sequence of an AAV-binding cyclic peptide into one or two superficial loops of the Fc portion of an antibody against a target receptor.
(17) In some embodiments, "the molecule" described in the present specification may be a Nanobody-Fc fusion protein obtained by fusing, via the hinge sequence of an antibody, module (B), which is a Nanobody against a target receptor, with module (A), which has acquired AAV-binding ability by incorporating, by the LassoGraft method, the internal sequence of an AAV-binding cyclic peptide into one or two superficial loops of an Fc fragment of the antibody.
(18) In some embodiments, "the molecule" described in the present specification may be an artificial protein that has acquired AAV-binding ability by incorporating, by the LassoGraft method, the internal sequence of an AAV-binding cyclic peptide into one or two superficial loops of serum albumin, and also acquired target receptor-binding ability by incorporating, by the LassoGraft method, the internal sequence of a target receptor-binding cyclic peptide into one or two superficial loops other than the above.
(19) In some embodiments, the "molecule" described in the present specification may be a complex obtained by reacting module (A), which is a biotinylated AAV-binding cyclic peptide, and module (B), which is a biotinylated target receptor-binding cyclic peptide, with streptavidin having four equivalent biotin-binding sites in an abundance ratio of 3:1, 2:2, or 1:3.
(20) In some embodiments, "the molecule" described in the present specification may be a complex obtained by reacting module (A), which is a biotinylated AAV-binding cyclic peptide, and module (B), which is a biotinylated scFv fragment of an antibody against a target receptor or an anti-target receptor nanobody, with streptavidin having four equivalent biotin-binding sites in an abundance ratio of 3:1, 2:2, or 1:3.
(21) In some embodiments, "the molecule" described in the present specification may be a protein in which module (A), which has acquired AAV-binding ability by incorporating, by the LassoGraft method, the internal sequence of an AAV-binding cyclic peptide into one or two of the surface loops of Fn10, is fused to the C-terminus or N-terminus of module (B) of an anti-target receptor antibody. Module (A) may be fused to either the H chain or L chain of the anti-target receptor antibody.

### [Use of "molecule" described in the present specification]

The "molecule" described in the present specification functions as an "engager" that brings AAV into proximity with cells by allowing a complex with a recombinant AAV containing a native AAV capsid to act on cells as a vector, thereby affording receptor-dependent gene transfer efficiency that is strikingly higher than when native AAV alone is used as a vector.

### (Preparation of complex of engager and recombinant AAV)

In an embodiment, an engager is added to AAV containing a capsid of a serotype whose genes have been recombined for the purpose of introducing a gene of interest ("recombinant AAV") to form a complex, which is then used as a "recombinant viral vector". The complex can be prepared, for example, in a test tube by adding a solution containing the engager to a solution containing the recombinant AAV, followed by incubation.

The mixing ratio of the engager and recombinant AAV in preparing the complex is determined appropriately depending on the characteristics of the engager and is not particularly limited. For example, a complex can be prepared by mixing recombinant AAV:engager at a ratio of 1:0.1 to 1:10 when converted to a ratio of (moles of capsid subunits):(moles of AAV capsid-binding peptide in module A).

### (Recombinant viral vector)

The recombinant viral vector described in the present specification includes a naturally occurring serotype AAV viral capsid, where the viral capsid encapsulates a nucleotide of interest. In some embodiments, the nucleotide of interest is under the control of a promoter selected from the group consisting of a viral promoter, a bacterial promoter, a mammalian promoter, an avian promoter, a fish promoter, an insect promoter, and any combination thereof. In some embodiments, the nucleotide of interest is under the control of a non-human promoter. In some embodiments, the promoter is a cytomegalovirus (CMV) promoter. In some embodiments, the promoter is an EF1α promoter. In some embodiments, the promoter is a CAG promoter.

The nucleotide of interest can be one or more genes, which can encode a detectable marker, for example, a reporter or a therapeutic polypeptide. In some embodiments, the nucleotide of interest is a reporter gene. In some embodiments, the nucleotide of interest is a reporter gene encoding a detectable marker selected from the group consisting of fluorescent protein, luciferase, β-galactosidase, and the like. In other embodiments, the nucleotide of interest is selected from the group consisting of a suicide gene, a nucleotide encoding an antibody or a fragment thereof, a nucleotide encoding a CRISPR/Cas system or a portion thereof, a nucleotide encoding an antisense RNA, a nucleotide encoding an siRNA, a secreted enzyme, a gene encoding a therapeutic protein, and the like. In some embodiments, the nucleotide of interest encodes a cyclic peptide-embedded grafted protein designed using the LassoGraft method.

The compositions described in the present specification generally contains "the molecule" described in the present specification and a viral capsid of a naturally occurring serotype, and the capsid encapsulates the nucleotide of interest. In some embodiments, the composition described in the present specification contains (1) a viral vector composed of a complex of "the molecule" described in the present specification and a viral capsid, and (2) a pharmaceutically acceptable carrier.

Methods for producing and using compositions composed of the above-mentioned "molecule" (hereafter referred to as "engager") as an engager and a viral capsid are also described in the present specification. In some embodiments, methods for retargeting adeno-associated viruses, delivering diagnostic/therapeutic cargo to target cells, and the like involve contacting a target cell (which may be in vitro or in vivo, e.g., in a human) with an engager-AAV capsid complex described in the present specification. The engager-AAV capsid complex coats the surface of the AAV capsid via module (A) in the engager, thereby preventing the AAV from attaching to nontarget cells, and at the same time, specifically binds to a protein expressed on the surface of the target cell via module (B), thereby achieving highly efficient infection and transduction of the target cell. Such methods may include steps such as a step of generating a viral vector having a native serotype capsid, for example, purifying a recombinant AAV vector containing a target nucleotide that has been recombinantly expressed using packaging cells; a step of chemically synthesizing an engager or recombinantly expressing and purifying an engager using a plasmid encoding the engager; and a step of mixing and incubating these two components in an appropriate ratio to obtain a complex.

In some embodiments, the target cell is a (human) hepatocyte, and module (B) of the engager contains an active structure that specifically binds to the asialoglycoprotein receptor (ASGR). In some embodiments, the target cell is a (human) neuron, and module (B) in the engager contains an active structure that specifically binds to a GABA receptor, TrkB, BACE1, SORL1, or the like. In some embodiments, the target cell is a (human) T cell, and module (B) in the engager contains an active structure that specifically binds to CD3, CD137, PD-1, CTLA-4, LAG-3, CD28, GITR, OX40, CD27, Thy-1, or the like. In some embodiments, the target cell is a (human) hematopoietic stem cell, and module (B) in the engager contains an active structure that specifically binds to CD34. In some embodiments, the target cell is a (human) brain endothelial cell, and module (B) in the engager contains an active structure that specifically binds to a Ly6 family molecule, transferrin receptor, carbonic anhydrase IV, CD98hc, insulin receptor, leptin receptor, or the like. In some embodiments, the target cell is a (human) muscle cell, and module (B) in the engager contains an active structure that specifically binds to an integrin, MuSK, LRP4, acetylcholine receptor, or the like. In some embodiments, the target cell is a (human) cancer cell, and module (B) in the engager contains an active structure that specifically binds to a tumor-associated antigen, such as the EGF receptor family, CD44, EpCAM, cMet, Plexin B1 or B2, or the like. In some embodiments, the target cell is a (human) Schwann cell, and module (B) in the engager contains an active structure that specifically binds to myelin protein zero (MPZ), myelin-associated glycoprotein (MAG), or the like. In some embodiments, the target cell is a (human) macrophage cell, and module (B) in the engager contains an active structure that specifically binds to SIRPα. In some embodiments, the target cell is a (human) microglial cell, and module (B) in the engager contains an active structure that specifically binds to TREM2. In some embodiments, the target cells are (human) retinal photoreceptor cells, and module (B) in the engager contains an active structure that specifically binds to peripherin 2 (PRPH2), ROM1, CD73, CD39, or the like. In some embodiments, the target cells are (human) Muller glial cells, and module (B) in the engager contains an active structure that specifically binds to GLAST (SLC1A3), CD44, or the like.

### (Embodiment of use of recombinant viral vector)

The recombinant viral vectors described in the present specification can be used for various therapeutic purposes (in vivo and in vitro) or as research tools, depending on the characteristics of the foreign gene introduced into the AAV.

When used for therapeutic purposes, the above-mentioned recombinant viral vectors can be used alone or in the form of pharmaceutical compositions formulated using one or more pharmaceutically acceptable carriers and/or excipients by methods known in the art (hereinafter collectively referred to as "the pharmaceutical composition"). Pharmaceutical compositions can be formulated for various modes of administration, including systemic administration, topical administration, and localized administration.

Pharmaceutical compositions can be administered to a subject (human or animal) in need thereof, for example, by injection, inhalation, or oral, buccal, parenteral, or rectal administration. More specifically, pharmaceutical compositions can be administered by any means known in the art. For example, pharmaceutical compositions can be administered to a subject intravenously, intratumorally, intradermally, intraarterially, intraperitoneally, intralesionally, intracranially, intraventricularly, intrathecally, intraarticularly, intraprostatically, intrapleurally, intratracheally, intranasally, intravitreally, intravaginally, intrarectally, topically, intratumorally, intramuscularly, intrathecally, subcutaneously, subconjunctivally, intravesicularly, mucosally, intrapericardially, intraumbilically, or intraocularly, orally, topically, by inhalation, injection, infusion, continuous infusion, and the like. The route of administration can be determined appropriately depending on the purpose of administration.

Systemic administration includes intramuscular, intravenous, intraperitoneal, and subcutaneous administration, and administration by injection is preferred. For injection, the pharmaceutical composition may be formulated in a liquid solution, preferably using a physiologically compatible buffer such as Hank's solution and Ringer's solution. In addition, the pharmaceutical composition may be formulated in a solid form and redissolved or suspended immediately prior to use for injectable administration. Lyophilized forms of the pharmaceutical composition are also suitable.

The pharmaceutical composition may be formulated for parenteral administration by injection, for example, bolus injection or continuous infusion. Formulations for injection may be provided in unit dosage form, for example, in ampoules, with optionally added preservatives. The pharmaceutical composition may be formulated as a suspension, solution, or emulsion in an oily or aqueous vehicle, and may contain other agents, including suspending, stabilizing, and/or dispersing agents.

For oral administration, pharmaceutical compositions can be prepared by conventional means in the art with pharmaceutically acceptable excipients, such as binders (e.g., pregelatinized maize starch, polyvinylpyrrolidone, or hydroxypropylmethylcellulose), fillers (e.g., lactose, microcrystalline cellulose, or calcium hydrogen phosphate), lubricants (e.g., magnesium stearate, talc, or silica), disintegrants (e.g., potato starch or sodium starch glycolate), or wetting agents (e.g., sodium lauryl sulfate), and can take the form of, for example, tablets or capsules. Tablets can also be coated by methods well known in the art. Liquid preparations for oral administration can take the form of, for example, solution, syrup, or suspension, or can be presented as a dry product for constitution with water or other suitable vehicle before use. Such liquid preparations can be prepared by conventional means using pharmaceutically acceptable additives such as suspending agents (e.g., sorbitol syrup, cellulose derivatives, or hydrogenated edible fats), emulsifying agents (e.g., lecithin or acacia), non-aqueous vehicles (e.g., ethanol oil, oily esters, ethyl alcohol, or fractionated vegetable oils), and preservatives (e.g., methyl or propyl-p-hydroxybenzoates or sorbic acid). Preparations can also contain buffer salts, flavoring agents, coloring agents, and sweetening agents, as appropriate.

Systemic administration can also be performed by transmucosal or transdermal means.

Pharmaceutical compositions suitable for injectable use can include sterile aqueous solutions or dispersions, formulations including sesame oil, peanut oil, or aqueous propylene glycol, and sterile powders for the extemporaneous preparation of sterile injectable solutions or dispersions.

When administering the above-mentioned pharmaceutical composition to a subject, one of ordinary skill in the art can determine the appropriate dosage and administration period for each individual subject, taking into consideration various factors such as the purpose of gene transfer, dosage form, administration route, the disease state of the subject, and the like.

When the above-mentioned recombinant viral vector is administered to a subject for therapeutic purposes, it can also be used in combination with other drugs (concomitant drugs). The need for combined use, the concomitant drugs, and their dosage and administration methods can be appropriately determined by one of ordinary skill in the art, whereby a combination therapy can be performed.

### [Example]

The embodiments of the present invention are described in detail below based on examples, but the present invention is not limited thereto. Those skilled in the art may modify the embodiments of the present invention in various ways without departing from the gist of the present invention, and such modifications are also within the scope of the present invention.

### Materials and Methods

### [Cell Lines]

Respective cell lines HEK293T, MDA-MB-231 (MM-231), bEND.3, and A498 were obtained from American Type Culture Collection (ATCC, Manassas, VA) and maintained in the media recommended by the ATCC. Expi293F cells were purchased from Thermo Fisher Scientific and maintained in the recommended media. The stable Plexin B1-expressing cell line described by Matsunaga et al., Cell Chem. Biol. 2016, was used. TrkB-expressing cell lines were established using Expi293F cells with reference to Matsunaga et al.

### [Production and purification of AAV viral vectors]

AAV viral vectors were produced using the AAVpro Heler-Free System manufactured by Takara Bio Inc. AAV9 capsids were produced using the plasmid pAAV2/9n (Addgene #112865), which contains the AAV2-derived Rep gene and the AAV9-derived Cap gene in tandem. The reporter gene used was pAAV-CMV-Fluc, obtained by cloning Firefly luciferase (Fluc) into the pAAV-CMV vector. Virus was produced by triple transfection of pHelper, pAAV2/9n, and pAAV-CMV-Fluc into HEK293T packaging cells by a conventional method.

The transfected packaging cells were cultured at 37°C in 5% CO₂ for 2 days, then harvested and resuspended in phosphatebuffered saline (PBS, 20 mM phosphate, 150 mM NaCl, pH 7.5) containing 1 mM MgCl₂, 2.5 mM KCl, and 0.5% Triton-X100. The cell suspension was frozen at -80°C for 30 min, thawed in a 37°C water bath, and centrifuged at 4000xg for 10 min at 4°C. This process was repeated three times, and finally centrifuged at 4000xg for 10 min at 4°C, after which the supernatant was collected. This was used as the virus extract.

The virus was purified by affinity chromatography using POROS^{™} CaptureSelect AAVX resin (Cat. No. A36739) manufactured by Thermo Fisher. Briefly, the virus extract was passed through a φ0.45 µm filter and passed through a POROS^{™} CaptureSelect AAVX resin column equilibrated with PBS. After washing away unbound material, the virus was eluted with elution buffer (40% propylene glycol, 0.1 M Na-citrate pH 3, 0.3 M NaCl). The eluted fraction was neutralized with 1/12 volume of 2 M HEPES pH 8. The obtained purified virus solution was replaced with PBS containing 0.001% Pluronic F68 polyole (MP Biomedical, Cat. No. 2750049) and concentrated by centrifugation to the desired volume using an Amicon Ultra-0.5 centrifugal filter unit with an Ultracel-100 membrane (100K Da MWCO) filter cartridge. The solution was then stored at 4°C or -80°C until use. The virus titer (viral genome vg/mL per milliliter) was determined by qPCR using a standard curve of virus with known concentration.

### [Preparation of Cyclic Peptides]

Purified AAV9 capsid was subjected to the RaPID system based on WO 2011/049157, JP 2013-46637 A, and the like to identify three classes of cyclic peptides that have high affinity and specifically bind to AAV9 capsid.

The identified cyclic peptides were chemically synthesized using methods well known in the art and used in the preparation of additives and other experiments. Methods for producing cyclic peptides include, for example, chemical synthesis methods such as the liquid-phase method, the solid-phase method, and a hybrid method combining the liquid-phase method and solid-phase method, gene recombinant method, translational synthesis method using a cell-free translation system, and the like.

Representative examples of the AAV9 capsid-binding cyclic peptides of classes 1 to 3 identified above are shown below.

### Example 1: Class 1 cyclic peptides

The active structure of representative Class 1 cyclic peptides (Examples 1-1 to 1-15) is a cyclic peptide represented by the following formula (Ia). The name of the cyclic peptide (L number) is also indicated for particularly representative peptides. 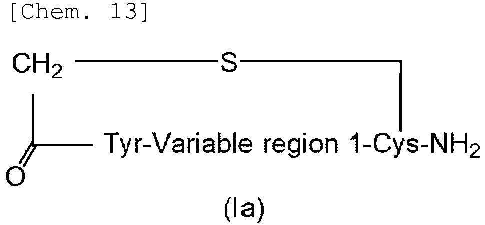 wherein Variable region 1 is the amino acid sequence of Variable region 1 in the cyclic peptide of each Example shown in Table 20 below, and S is S derived from the thiol group of Cys.

**[Table 20]**

| Example No. | amino acid sequence of Variable region 1 (including single letter notation) | SEQ ID NO: |
|---|---|---|
| 1-1 (L1) | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |
| 1-6 | Thr Tyr Arg Thr Thr Ile Pro His Glu Tyr Leu Asp(TYRTTIPHEYLD) | 24 |
| 1-7 | Thr Tyr Ser Thr Thr Ile Pro His Glu Tyr Leu Ala(TYSTTIPHEYLA) | 25 |
| 1-8 | Thr Tyr Arg Thr Thr Ile Pro Tyr Glu Tyr Leu Ala(TYRTTIPYEYLA) | 26 |
| 1-9 | Thr Tyr His Thr Thr Ile Pro His Glu Tyr Leu Ala(TYHTTIPHEYLA) | 27 |
| 1-10 (L17) | Thr Tyr Arg Asn Thr Ile Pro His Glu Tyr Leu Ala(TYRNTIPHEYLA) | 28 |
| 1-11 | Asn Tyr Arg Thr Thr Ile Pro His Glu Tyr Leu Ala(NYRTTIPHEYLA) | 29 |
| 1-12 | Ile Tyr Arg Thr Thr Ile Pro His Glu Tyr Leu Ala(IYRTTIPHEYLA) | 30 |
| 1-13 | Thr Tyr Arg Thr Thr Ile Pro Pro Glu Tyr Leu Ala(TYRTTIPPEYLA) | 31 |
| 1-14 | Thr Tyr Arg Thr Thr Ile Pro His Glu Tyr Leu Val(TYRTTIPHEYLV) | 32 |
| 1-15 | Thr Tyr Arg Thr Thr Ile Pro His Glu Tyr Leu Thr(TYRTTIPHEYLT) | 33 |

### Example 2: Class 2 cyclic peptides

The active structure of representative Class 2 cyclic peptides (Examples 2-1 to 2-15) is a cyclic peptide represented by the following formula (Ib). The name of the cyclic peptide (L number) is also indicated for particularly representative peptides. 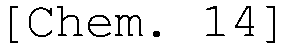 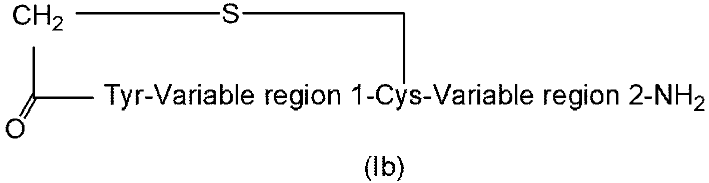 wherein Variable region 1 is the amino acid sequence of Variable region 1 in the cyclic peptide of each Example shown in Table 21 below, Variable region 2 is the amino acid sequence of Variable region 2 in the cyclic peptide of each Example shown in Table 22 below, and S is S derived from the thiol group of Cys.

**[Table 21]**

| Example No. | amino acid sequence of Variable region 1 (including single letter notation) | SEQ ID NO: |
|---|---|---|
| 2-1 (L2) | Ser Lys Ser Val (SKSV) | 9 |
| 2-2 | Ser Lys Ser Val (SKSV) | 9 |
| 2-3 | Ser Lys Ser Val (SKSV) | 9 |
| 2-4 | Ser Lys Ser Val (SKSV) | 9 |
| 2-5 | Ser Lys Ser Val (SKSV) | 9 |
| 2-6 | Ser Lys Ser Val (SKSV) | 9 |
| 2-7 | Ser Lys Ser Val (SKSV) | 9 |
| 2-8 | Ser Lys Ser Val (SKSV) | 9 |
| 2-9 | Ser Lys Ser Val (SKSV) | 9 |
| 2-10 | Ser Lys Ser Val (SKSV) | 9 |
| 2-11 | Ser Lys Ser Val (SKSV) | 9 |
| 2-12 | Ser Lys Ser Val (SKSV) | 9 |
| 2-13 | Ser Lys Ser Val (SKSV) | 9 |
| 2-14 | Ser Lys Ser Ile (SKSI) | 11 |
| 2-15 | Ser Lys Ser Val (SKSV) | 9 |

**[Table 22]**

| Example No. | amino acid sequence of Variable region 2(including single letter notation) | SEQ ID NO: |
|---|---|---|
| 2-1 (L2) | Leu Ala Gln Asp Trp Glu Tyr Lys Trp (LAQDWEYKW) | 14 |
| 2-2 | Leu Ala Gln Asp Trp Glu Tyr Lys (LAQDWEYK) | 15 |
| 2-3 | Leu Ala Lys Asp Trp Glu Tyr Lys Trp (LAKDWEYKW) | 16 |
| 2-4 | Leu Ala Gln Asp Trp Val Tyr Lys Tyr (LAQDWVYKY) | 17 |
| 2-5 | Leu Ala Gln Asp Trp Lys Tyr Lys Trp (LAQDWKYKW) | 18 |
| 2-6 | Leu Ala Gln Asp Trp Glu Tyr Glu Trp (LAQDWEYEW) | 34 |
| 2-7 | Leu Ala Gln Asp Trp Glu Tyr Leu Ala (LAQDWEYLA) | 35 |
| 2-8 | Leu Ala Gln Asp Trp Glu Tyr Arg Trp (LAQDWEYRW) | 36 |
| 2-9 | Leu Ala Gln Asn Trp Glu Tyr Lys Trp (LAQNWEYKW) | 37 |
| 2-10 | Leu Ala Gln Asp Trp Asp Tyr Lys Trp (LAQDWDYKW) | 38 |
| 2-11 | Leu Ala Lys Asp Trp Glu Tyr Lys (LAKDWEYK) | 39 |
| 2-12 | Leu Thr Gln Asp Trp Glu Tyr Lys Trp (LTQDWEYKW) | 40 |
| 2-13 | Leu Ala Gln Asp Trp Glu Tyr Asn Trp (LAQDWEYNW) | 41 |
| 2-14 | Leu Ala Gln Asp Trp Glu Tyr Lys Trp (LAQDWEYKW) | 42 |
| 2-15 | Leu Ala Gln Asp Trp Glu Tyr Lys Arg (LAQDWEYKR) | 43 |

### Example 3: Class 3 cyclic peptides

The active structure of representative Class 3 cyclic peptides (Examples 3-1 to 3-8) is a cyclic peptide represented by the following formula (Ic). The name of the cyclic peptide (L number) is also indicated for particularly representative peptides. 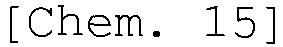 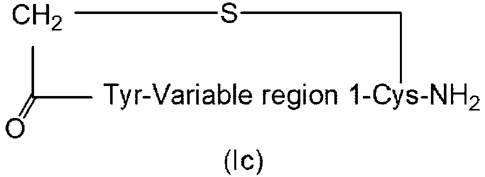 wherein Variable region 1 is the amino acid sequence of Variable region 1 in the cyclic peptide of each Example shown in Table 23 below and S is S derived from the thiol group of Cys.

**[Table 23]**

| Example No. | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 (L19) | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |
| 3-6 | Arg Phe Tyr Lys Ser Leu His Leu Ala Gln Gln Tyr Gln Arg Ile (RFYKSLHLAQQYQRI) | 44 |
| 3-7 | Arg Phe Ser Lys Ser Leu His Ile Ala Gln Gln Tyr Gln Arg Ile (RFSKSLHIAQQYQRI) | 45 |
| 3-8 | Arg Phe Ser Lys Ser Phe His Leu Ala Gln Gln Tyr Gln Arg Ile (RFSKSFHLAQQYQRI) | 46 |

### [Various embodiments as engager]

The core of the technique of the present invention is an engager molecule that attracts native AAV capsids to desired receptor-expressing cells, i.e., a "molecule" containing an AAV capsid-binding module (A) and a target receptor-binding module (B), which can simultaneously bind to the AAV capsid and the target receptor, and can provide embodiments of an extremely diverse range of engagers. Representative embodiments include (a) heterogenous cyclic peptide conjugate (referred to as type I engager), (b) cyclic peptide-target antibody conjugate (referred to as type II engager), (c) conjugate of two scaffold proteins grafted with cyclic peptides (referred to as type III engager), (d) single scaffold protein grafted with multiple cyclic peptides (referred to as type IV engager), (e) anti-target receptor antibody grafted with a cyclic peptide (referred to as type V engager), (f) conjugate of an anti-target receptor and a scaffold protein grafted with a cyclic peptide (referred to as type VI engager), and the like.

These embodiments are shown in Fig. 1.

In the following, various embodiments using the above-mentioned "molecules" as engagers are described using examples. However, these are examples for specific understanding of the various embodiments, and respective embodiments are not limited to these.

### <Preparation of engagers>

### Example 4: Preparation of heterogeneous cyclic peptide conjugate (type I engager)

The molecule in which the AAV capsid-binding peptide and the target receptor-binding peptide were directly linked was prepared as follows.

First, cyclic peptides biotinylated via a Lys residue added to the C-terminus were synthesized. These were then incubated with streptavidin at room temperature for 30 min in an appropriate molar ratio to obtain a streptavidin:peptide complex that presented both peptides in an appropriate ratio. For example, by setting the mixing ratio of AAV capsid-binding peptide:target receptor-binding peptide:streptavidin to 3:1:1, a complex was prepared in which one molecule of streptavidin presented the AAV capsid-binding peptide in a trivalent form and the target receptor-binding peptide in a monovalent form.

### Example 5: Preparation of cyclic peptide-target antibody conjugate (type II engager)

The molecule in which the AAV capsid-binding peptide and anti-target receptor antibody were directly linked was prepared as follows. First, an expression vector was created by fusing the gene for a single-chain type antibody (scFv) against the target receptor with biotin-acceptor sequence (BAS). This was transfected into Expi293F cells together with an expression vector for the biotin ligase BirA to obtain biosynthetically biotinylated anti-receptor scFv. This biotinylated scFv and a chemically synthesized biotinylated AAV capsid-conjugated cyclic peptide were incubated with streptavidin in an appropriate molar ratio at room temperature for 30 min to obtain a ternary complex. For example, by setting the mixing ratio of AAV capsid-binding peptide:target receptor-binding scFv:streptavidin to 3:1:1, a complex was prepared in which one molecule of streptavidin presented the AAV capsid-binding peptide in a trivalent form and the target receptor-binding peptide in a monovalent form.

### Example 6: Preparation of conjugate of two scaffold proteins grafted with cyclic peptides (type III engager)

A method for producing scaffold proteins grafted with AAV capsid-binding peptides and target receptor-binding peptides by using the LassoGraft method is described in detail in Japanese Patent No. 6598344. Furthermore, by genetically fusing the two, molecules that bind to both the AAV capsid and the target receptor can be prepared.

As one example, a molecule was prepared here as follows by linking a module in which an AAV capsid-binding peptide was grafted into the 10th Type III repeat domain of human fibronectin (hereinafter referred to as "Fn10") with a module in which a target receptor-binding peptide was grafted into the human IgG-derived Fc region (hereinafter referred to as "Fc").

An Fn10:Fc fusion protein construct was produced by connecting DNA encoding the signal sequence of human prolactin, DNA encoding the region of human fibronectin from amino acid residue numbers 1418 to 1509, and DNA encoding the Fc region of human IgG1. This construct was then incorporated into the expression vector pcDNA3.1 (manufactured by Thermo Fisher Scientific). Based on the Fn10:Fc expression vector, an expression vector was produced in which one amino acid residue in the loop within the fibronectin region, for example, the loop region between Pro1440 and Val1442, was replaced with a sequence derived from the AAV9 capsid-binding cyclic peptide shown in Table 24, and one amino acid residue in the loop within the Fc region, for example, the loop region between Ser383 and Gly385, was further replaced with a sequence derived from the target receptor-binding cyclic peptide shown in Table 25. The grafted sequence portion was amplified using extension PCR to obtain constructs of various target type III engagers. This expression construct was used to transiently express the target protein using an Expi293 Expression System (manufactured by Thermo Fisher Scientific), and the target protein was purified from the culture supernatant by affinity chromatography using Protein A-Sepharose (manufactured by Cytiva). The purified type III engager protein was dialyzed against PBS and stored at -80°C until use. The cyclic peptide-grafted proteins were indicated as Fn10 (cyclic peptide name):Fc (cyclic peptide name), for example, Fn10(L19):Fc(PB1m6).

**[Table 24]**

| Example No. | name | amino acid sequence (including single letter notation) | SEQ ID NO: |
|---|---|---|---|
| 6-1 | L1 | Ser Gly Gly Gly Cys Tyr Thr Tyr Arg Thr Thr Ile Pro His Glu Tyr Leu Cys Gly Gly Gly Ser (SGGGCYTYRTTIPHEYLCGGGS) | 47 |
| 6-2 | L19 | Ser Gly Gly Gly Ser Gly Cys Tyr Arg Phe Ser Lys Ser Leu His Leu Ala Gln Glu Tyr Gln Arg Ile Cys Gly Gly Ser Gly Gly Ser (SGGGSGCYRFSKSLHLAQEYQRICGGSGGS) | 48 |
| 6-3 | L17 | Ser Gly Gly Gly Cys Tyr Thr Tyr Arg Asn Thr Ile Pro His Glu Tyr Leu Cys Gly Gly Gly Ser (SGGGCYTYRNTIPHEYLCGGGS) | 54 |

**[Table 25]**

| Example No. | name | target molecule | amino acid sequence (including single letter notation) | SEQ ID NO: |
|---|---|---|---|---|
| 6-A | PB1m6 | PlexinB1 | Ser Gly Trp Arg Pro Arg Val Ala Arg Trp Thr Gly Gln Ile Ile Tyr Gly Ser (SGWRPRVARWTGQIIYGS) | 49 |
| 6-B | A6-2f | EGFR | Ser Gly Gly Cys Ala Ser Phe Pro Glu Glu Leu Asp Glu Trp Tyr Val Ala Tyr Gly Cys Gly Gly Ser (SGGCASFPEELDEWYVAYGCGGS) | 50 |
| 6-C | 44D8 | CD44 | Ser Gly Gly Tyr Val Glu Pro Ile Val Thr Arg Tyr Ala Ile Tyr Trp Lys Leu Gly Gly Ser (SGGYVEPIVTRYAIYWKLGGS) | 51 |
| 6-D | tkL3 | TrkB | Ser Gly Cys Tyr Leu Arg Pro Arg Tyr Lys Phe Trp Cys Gly Gly Ser (SGCYLRPRYKFWCGS) | 52 |
| 6-E | L6L41 | Ly6c1 | Ser Gly Gly Tyr Thr Gly His Thr Trp Thr Leu Asn Ser Gly Tyr Arg Gly Gly Ser (SGGYTGHTWTLNSGYRGGS) | 53 |

### Example 7: Preparation of single scaffold protein grafted with multiple cyclic peptides (type IV engager)

As one example of a type IV engager, a case where human serum albumin (hereinafter referred to as "HSA") is selected as the scaffold protein is described here. An HSA fusion protein construct was created by ligating DNA encoding full-length HSA, DNA encoding a Hisx8 tag, and DNA encoding a Myc tag, and then incorporated into the expression vector pcDNA3.1 (manufactured by Thermo Fisher Scientific). Based on the HSA expression vector, an expression vector was produced in which one amino acid residue in the loop region between, for example, Ala171 and Asp173, and one amino acid residue between Ala561 and Asp563, were replaced with a sequence derived from the AAV9 capsid-binding cyclic peptide shown in Table 24, and one amino acid residue in the loop region between Ala363 and Asp365 was further replaced with a sequence derived from the target receptor-binding cyclic peptide shown in Table 25. The grafted sequence portion was amplified using extension PCR to obtain constructs of various cyclic peptide fusion proteins. This expression construct was used to transiently express the target protein using an Expi293 Expression System (manufactured by Thermo Fisher Scientific), and the target protein was purified from the culture supernatant by metal chelate chromatography using Ni-NTA agarose (manufactured by Qiagen). The purified type IV engager protein was dialyzed against PBS and stored at -80°C until use. The cyclic peptide-grafted proteins were each indicated as HSA (first cyclic peptide name) (valency) (second cyclic peptide name) (valency), for example, HSA(L19)₂(44D8)₁.

### Example 8: Preparation of anti-target receptor antibody (type V engager) grafted with cyclic peptide

The technique of conferring new binding specificity to any antibody by using the LassoGraft method is described in detail in Japanese Patent No.7303527. Here, the original anti-receptor antibody used was an antibody with the constant region of human IgG1/kappa and the variable region of the anti-human CD44 antibody H460 or the anti-mouse transferrin receptor antibody 8D3. The amino acid sequences of the variable regions of H460 and 8D3 were those described in US Patent No. 8,071,072 and Boado et al., Biotech Bioeng. 2008, respectively. Based on the antibody heavy chain expression vector, an expression vector was produced in which one amino acid residue in the loop region between, for example, Tyr296 and Ser298, and one amino acid residue in the loop region between Ser383 and Gly385, were replaced with a sequence derived from the AAV9 capsid-binding cyclic peptide shown in Table 24. The grafted sequence portion was amplified using extension PCR to obtain heavy chain constructs of various type V engagers. This expression construct was used together with the corresponding light chain expression vector to transiently express the target bispecific antibody using an Expi293 Expression System (manufactured by Thermo Fisher Scientific), and the target protein was purified from the culture supernatant by affinity chromatography using Protein A-Sepharose (manufactured by Cytiva). The purified type V engager protein was dialyzed against PBS and stored at - 80°C until use. The cyclic peptide-grafted proteins were each indicated as (antibody name)-IgG (cyclic peptide name) (valency), for example, H460-IgG(L19)₂.

### Example 9: Preparation of conjugate of scaffold protein grafted with cyclic peptide and anti-target receptor antibody (type VI engager)

A method for producing a scaffold protein grafted with an AAV capsid-binding peptide by using the LassoGraft method is described in detail in Japanese Patent No. 6,598,344. Furthermore, by genetically fusing this to any antibody, a molecule that binds to both the AAV capsid and a target receptor can be prepared.

As one example here, a molecule in which a module grafted with an AAV capsid-binding peptide into Fn10 was fused to the C-terminus of an antibody against a target receptor was prepared as follows.

Here, the original anti-receptor antibody used was an antibody with the constant region of human IgG1/kappa and the variable region of the anti-human HER2 antibody Trastuzumab or the anti-mouse transferrin receptor antibody 8D3. The variable region amino acid sequences of trastuzumab and 8D3 used were those described in DRUGBANK (https://go.drugbank.com/drugs/DB00072) and Boado et al., Biotech Bioeng. 2008, respectively. Heavy chain constructs of the desired various type VI engagers were obtained by linking Fn10 module to the C-terminus of the antibody heavy chain, and replacing one amino acid residue in the loop region between, for example, Pro1440 and Val1442 with a sequence derived from the AAV9 capsid-binding cyclic peptide shown in Table 24. This expression construct was used together with the corresponding light chain expression vector to transiently express the target antibody-Fn10 fusion product by using an Expi293 Expression System (manufactured by Thermo Fisher Scientific), and the target protein was purified from the culture supernatant by affinity chromatography using Protein A-Sepharose (manufactured by Cytiva). The purified type VI engager protein was dialyzed against PBS and stored at -80°C until use. The antibody-Fn10 fusion products were each indicated as (antibody name):Fn10 (cyclic peptide name), for example, Trastuzumab:Fn10(L17).

### <Cell transduction with AAV9 viral particles using engagers>

### Example 10: Preparation of targeted AAV9 vector complex and cell infection and transduction

Purified viral particles were mixed with various engagers and incubated at room temperature for 30 min to form complexes. Since one AAV9 viral particle contains 60 Cap subunits, each of which can bind one capsid-binding cyclic peptide, a molar ratio of 60:1 between engagers and recombinant AAV9 particles is theoretically equivalent. Based on this, engagers were mixed at three concentrations (0.1, 1, and 10 equivalents) relative to the virus, and cells were infected with these. Cells for infection and transduction evaluation were seeded at 5,000 cells/well in a 96-well Black Clear plate (manufactured by Greiner, #655090) and cultured in DMEM medium containing 5% FCS under the conditions of 6 hr at 37°C and 5% CO₂. Targeted AAV9 vector complex was added to each well to a viral titer of 5x10⁸ vg/well, and the cells were cultured under the conditions of 37°C and 5% CO₂ for 2 days. Luciferase expression was then measured using Luciferase Assay System (manufactured by Promega, Cat. No. E1501). Briefly, after removing the medium from the well using an aspirator, 200 µl of PBS was added. This was then removed, and 20 µl of lysis buffer was added. The cells were lysed by shaking at 1000 rpm for 20 min. 40 µl of substrate was then added, and the luminescence intensity was measured using a Promega Glo-Max luminometer.

### <Study of properties of cyclic peptide>

### Example 11

One representative cyclic peptide each from classes 1 to 3 was selected and chemically synthesized to have the structure shown in Fig. 2a (L1, L2, and L19). Their binding affinity to AAV9 capsid was analyzed by surface resonance plasmon resonance (SPR). As shown in Figs. 2b to 2d, the dissociation constants (KD) were all near 100 nM.

### Example 12

To identify the binding sites of the above-mentioned three representative cyclic peptides to the AAV9 capsid, the three-dimensional structure of the peptide-capsid complexes was analyzed by cryo-electron microscopy. Purified AAV9 capsid was mixed with 500 µM cyclic peptide to prepare cryo-samples. Images were then taken using a Glacios Cryo-TEM (manufactured by Thermo Fisher Scientific) at an accelerating voltage of 200 kV. Single-particle analysis was used to determine the respective atomic structures of the L1 peptide complex at a resolution of 2.48 Å, the L2 peptide complex at a resolution of 2.12 Å, and the L19 peptide complex at a resolution of 2.54 Å. As shown in Figs. 3a to 3c, 60 peptide molecules, the same number as the number of capsid subunits, bound per virus particle. L1 was found to bind near the two-fold symmetry axis of the virus, and L2 and L19 were found to bind near the threefold symmetry axis. As predicted from the sequence homology thereof, L2, which is a class 2 peptide, and L19, which is a class 3 peptide, bound to the same pocket on the capsid in a very similar conformation. Furthermore, when the binding sites of these peptides were overlaid on a planar projection of the amino acid residues of the capsid subunits on the viral surface, as shown in Fig. 3(d), none of them overlapped with the binding region of the AAVR, which is an internalization receptor essential for viral infection.

### Example 13

The three representative AAV9 capsid-binding cyclic peptides synthesized above were examined for the inhibitory effects on the ability of AAV9 to infect and transduce cells. Cyclic peptides equivalent to 0.1 equivalent, 1 equivalent, 10 equivalents, or 50 equivalents of capsid subunits (60 of which form one virus particle) were added to purified AAV9, HEK293 cells were infected with the peptide-AAV9 vector complex, and luciferase expression level was quantified. As shown in Fig. 4, none of the L1, L2, and L19 peptides showed attenuation of transduction compared to when no peptide was added, even when a large excess of 50 equivalents was added. This was consistent with the finding that all peptides bind to sites that do not compete with the infection receptor during virus recognition, as shown in Example 12.

### <Study of receptor-specific gene transfer ability by engagers>

### Example 14

To investigate the acquisition of receptor-specific gene transfer ability by type I engagers, streptavidin which contained three AAV9 capsid-binding peptides L1 (Fig. 2a, compound No. 1-1) and one Plexin B1-binding peptide PB1m6 (Matsunaga et al, Cell Chem. Biol. 2016) per molecule (referred to as (L1)₃(PB1m6)₁-SA) was prepared. This was added to purified AAV9 at an amount of 0.1 equivalent, 1 equivalent, or 10 equivalents per capsid subunit to form a complex. The complex was then allowed to act on Expi293F cells that do not express Plexin B1 or Expi293F cells that overexpress Plexin B1, and the luciferase expression level was quantified.

As shown in Fig. 5, this complex showed a concentration-dependent decrease in infectivity in cells that did not express Plexin B1. When 10 equivalents were added, about 40% inhibition of transduction was found compared to AAV9 alone. Since no inhibition of infectivity was observed even when 50 equivalents of the cyclic peptide L1 were added, it is considered that by making L1 a type I engager, a bulk derived from streptavidin (molecular weight 53 kDa) was gained, resulting in partial inhibition of the interaction between AAV and attachment receptors and endogenous receptors through steric hindrance.

On the other hand, in cells with forcible expression of Plexin B1, not only was there observed no inhibition of infectivity, but the addition of 10 equivalents of type I engager showed about five times higher transduction efficiency compared to AAV9 alone. Thus it was clarified that this method can increase the infectivity and transduction ability of AAV in a manner dependent on specific target receptors.

### Example 15

To investigate the acquisition of receptor-specific gene transfer ability by type II engagers, streptavidins which contained three AAV9 capsid-binding peptides L1 (Fig. 2a, compound No. 1-1) and one target receptor antibody scFv (anti-human CD44 antibody H460 and anti-mouse transferrin receptor (TfR) antibody 8D3) per molecule (referred to as L1:scFv(H460)-SA and L1:scFv(8D3)-SA) were prepared. This was added to purified AAV9 at an amount of 0.1 equivalent, 1 equivalent, or 10 equivalents per capsid subunit to form a complex which was was then allowed to act on various cells, and the luciferase expression level was quantified. As shown in Fig. 6, neither type II engager showed a significant decrease or increase in infectivity for HEK cells that do not express CD44 or TfR.

On the other hand, L1:scFv(H460)-SA increased the transduction ability of AAV9 in a concentration-dependent manner in human cancer-derived MM-231 cells that highly express CD44, while L1:scFv(8D3)-SA showed a 2- to 7-fold increase in transduction ability in mouse brain vascular endothelial cell line bEND.3 that expresses TfR. In both cells, when a type II engager containing scFv for a target receptor not expressed by the cell itself was added, the transduction activity tended to be equivalent to or rather inhibited compared to the addition of AAV9 alone.

### Example 16

To investigate the acquisition of receptor-specific gene transfer ability by type III engagers, an AAV9 capsid-binding peptide L19 (Table 24, Example No. 6-2) was grafted into Fn10, and cyclic peptide-derived sequences that bind to various target receptors (Table 25, Examples Nos. 6-A to 6-E) were transplanted into Fc to produce an Fn10:Fc fusion protein. This was added to purified AAV9 at an amount of 0.1 equivalent, 1 equivalent, or 10 equivalents per capsid subunit to form a complex which was then allowed to act on various cells, and the luciferase expression level was quantified.

As shown in Fig. 7a, the complex of AAV9 and type III engager grafted with Plexin B1-binding PB1m6 (Table 25, Example No. 6-A), showed a concentration-dependent decrease in infectivity in cells that did not express Plexin B1. However, in cells that forcibly expressed Plexin B1, the complex showed up to about seven times higher transduction efficiency compared to AAV9 alone. Similarly, type III engager grafted with EGFR-binding A6-2f (Table 25, Example 6-B) showed up to 3.5-fold increase in transduction efficiency in EGFR-highly expressing human cancer cells A498; type III engager grafted with CD44-binding 44D8 (Table 25, Example 6-C) showed up to 11-fold increase in CD44-highly expressing human cancer cells MM-231; and type III engager grafted with TrkB-binding tkL3 (Table 25, Example 6-D) showed up to 4.5-fold increase in transduction efficiency in Expi293F cells forcibly expressing TrkB. On the other hand, they showed concentration-dependent inhibition of infectivity in Expi293F or HEK cells that did not express respective receptors (Figs. 7b to 7d).

### Example 17

To investigate the effectiveness of type III engagers, and to determine whether AAV9 capsid-binding peptides other than class 3 peptides (L19) could also be used, L17 belonging to class 1 (Table 24, Example No. 6-3) was grafted into Fn10, and cyclic peptide-derived sequences that bind to various target receptors (Table 25, Example Nos. 6-B, 6-E) were transplanted into Fc to produce an Fn10:Fc fusion protein. This was added to purified AAV9 at an amount of 0.1 equivalent, 1 equivalent, or 10 equivalents per capsid subunit to form a complex which was was then allowed to act on various cells, and the luciferase expression level was quantified.

As shown in Fig. 8a, the complex of AAV9 and L17 graft-type III engager grafted with Ly6c1-binding L6L41 (Table 25, Example No. 6-E) did not affect the infectivity of HEK cells that do not express Ly6c1. However, in Ly6c1-expressing mouse cerebral vascular endothelial cell line bEND.3, the complex caused a dramatic, concentration-dependent increase in infectivity, showing up to 120 times higher transduction efficiency compared to AAV9 alone. Similarly, L17 graft-type III engager grafted with EGFR-binding A6-2f (Table 25, Example No. 6-B) showed up to 8 times higher transduction efficiency in EGFR-highly expressing human cancer cells A498, but only showed a very weak, concentration-dependent inhibition of infectivity in HEK cells that do not express EGFR (Fig. 8, Fig. 10b).

### Example 18

To investigate the acquisition of receptor-specific gene transfer ability by type IV engager, HSAs grafted by two AAV9 capsid-binding peptides L19 (Table 24, Example No. 6-2) and one target receptor-binding peptide (CD44-binding 44D8 (Table 25, Example No. 6-C) or Ly6c1-binding L6L41 (Table 25, Example No. 6-E)) per molecule (referred to as HSA(L19)₂(44D8)₁ and HSA(L19)₂(L6L41)₁) were produced. This was added to purified AAV9 at an amount of 0.1 equivalent, 1 equivalent, or 10 equivalents per capsid subunit to form a complex which was then allowed to act on various cells, and the luciferase expression level was quantified.

As shown in Fig. 9, both type IV engagers showed a concentration-dependent decrease in infectivity in HEK cells that do not express CD44 or Ly6c1, and almost completely lost the transduction ability when 10 equivalents were added.

On the other hand, HSA(L19)₂(44D8)₁ showed up to 10-fold increase in transduction effects compared to AAV9 alone, in human cancer-derived MM-231 cells that highly express CD44, and HSA(L19)₂(L6L41)₁ showed up to 13-fold increase in transduction effects compared to AAV9 alone, in mouse brain vascular endothelial cell line bEND.3 that expresses Ly6c1.

### Example 19

To investigate the acquisition of receptor-specific gene transfer ability by type V engagers, AAV9 capsid-binding peptide L19 (Table 24, Example No. 6-2) was grafted into two locations in the Fc region of target receptor antibodies IgG (anti-human CD44 antibody H460 and anti-mouse transferrin receptor (TfR) antibody 8D3) to produce bispecific antibodies (referred to as H460 IgG(L19)₂ and 8D3 IgG(L19)₂). This was added to purified AAV9 at an amount of 0.01 equivalent, 0.1 equivalent, 1 equivalent, or 10 equivalents per capsid subunit to form a complex which was then allowed to act on various cells, and the luciferase expression level was quantified.

As shown in Figs. 10a and 10b, both type V engagers caused a concentration-dependent reduction in infectivity in HEK cells that do not express CD44 or TfR.

On the other hand, H460 IgG(L19)₂ showed extremely high increase in transduction activity (17-fold and 57-fold) when added to human cancer-derived MM-231 cells that highly express CD44, at doses of 0.01 and 0.1 equivalent, respectively. However, when the added amount was increased to 1 equivalent, the transduction activity was limited to 6.6 times, and when 10 equivalents were added, it became about 1/10 of the amount without addition, suggesting that a strong inhibitory effect exists when added at high concentrations that counteracts the promoting effect. Similar results were also observed with 8D3 IgG(L19)₂, where AAV9 added at 0.01 equivalent, 0.1 equivalent, 1 equivalent, and 10 equivalents, showed 40-fold, 60-fold, 3.8-fold, and 0.16-fold transduction activity compared to AAV9 alone in mouse brain vascular endothelial cell line bEND.3 expressing TfR.

Based on these results, two type V engagers were fixed at an amount added of 0.1 equivalent and a complex was formed with AAV9. The complex was allowed to act on three types of cells, and luciferase activity was measured. As a result, 80-90% of transduction ability compared to AAV9 alone was shown in cells that did not express the target receptor of the grafted cyclic peptide, while transduction of 3000-8000% was observed in cells that express the target receptor (Fig. 10c).

### Example 20

To investigate the acquisition of receptor-specific gene transfer ability by type VI engager, AAV9 capsid-binding peptide L17 (Table 24, Example No. 6-3) was grafted into the C-terminus of target receptor antibodies IgG (anti-human HER2 antibody Trastuzumab and anti-mouse transferrin receptor (TfR) antibody 8D3) to produce antibody-Fn10 fusions having an Fn10 module (referred to as Trastuzumab:Fn10(L17) and 8D3:Fn10(L17)). This was added to purified AAV9 at an amount of 0.01 equivalent, 0.1 equivalent, 1 equivalent, or 10 equivalents per capsid subunit to form a complex which was was then allowed to act on various cells, and the luciferase expression level was quantified.

As shown in Figs. 11a and 11b, both type VI engagers caused almost no effect at any concentration on the infectivity of HEK cells that do not express HER2 or TfR.

On the other hand, Trastuzumab:Fn10(L17) showed an extremely high concentration-dependent increase in transduction ability (up to 285 times) in human cancer-derived SKBR3 cells that highly express HER2, and 8D3:Fn10(L17) showed a concentration-dependent increase in transduction ability of up to 12 times in mouse brain vascular endothelial cell line bEND.3 that expresses TfR. Interestingly, while type V engagers using antibodies against target receptors showed a strong inhibitory effect that counteracted the promoting effect when added at high concentrations, exhibiting a biphasic effect on transduction ability, type VI engagers did not show such pattern and exhibited the property that the higher the concentration, the greater the receptor-specific transduction ability.

### <Study of receptor-specific gene transfer ability by engager in animal>

### Example 21

To confirm whether the AAV-engager complex can exhibit directivity to the target receptor not only in vitro but also in vivo, administration to mice and tissue-dependent gene expression pattern were investigated. In this study, an L17 graft type III engager grafted with Ly6c1-binding L6L41 (Table 25, Example No. 6-E) described in Example 14.5 was used as the engager, and wild-type AAV9 particles carrying a luciferase reporter were used as the virus. Since Ly6c1 is specifically expressed in brain microvascular endothelial cells, it is expected that AAV9 coated with this engager will pass through the blood-brain barrier via receptor-mediated transcytosis and introduce genes into nerve cells in the brain parenchyma. The above-mentioned AAV9 and engager were mixed in an equivalent ratio of 1:10 and administered to ICR mice via tail vein at a dose of 1.5x10¹⁰ vg/mouse. Seven days after infection, live animals were applied to an IVIS spectrum in vivo imaging system (PerkinElmer) under isoflurane anesthesia to image the luminescence of luciferase. As shown in Fig. 12a, in AAV9 without the engager, luciferase expression was observed throughout the body, but the brain-specific luminescence in the head was not observed. Furthermore, AAV9 complexed with the control engager (single-specific protein that binds to AAV9 via L17 but lacks receptor binding ability) showed drastically attenuated infectivity due to competition with the infection receptor, and reporter expression was no longer observed in tissues throughout the body (Fig. 12b). In contrast, AAV9 complexed with a Ly6c1-binding type III engager showed low systemic luminescence, similar to the control, but exhibited strong reporter expression in the head, thus strongly suggesting crossing of the blood-brain barrier and successfully-achieved transduction into brain parenchymal cells.

As demonstrated above, it was clarified that, in all embodiments, the AAV-cell engager provided as one embodiment of the present invention affords an extremely superior transduction effect on cells expressing the target receptor, even though a native (unmodified) AAV capsid is used, and that this effect is demonstrated not only in vitro but also in vivo. When gene therapy is performed using the "molecule" described in the present specification on a target patient, for example, the conditions of usage and dosage, such as the amount of the "molecule" to be used as an engager for AAV, can be appropriately set by those skilled in the art.

### [Industrial Applicability]

The present invention relates to a method for conferring target molecule specificity to AAV, and provides a bivalent molecule capable of binding to both native AAV capsid and molecular target, as one embodiment of the invention. This molecule is useful, for example, in the field of gene therapy and the like.

This application is based on a patent application No. 2023-179190 filed in Japan (filing date: October 17, 2023), the contents of which are incorporated in full herein.

## Claims

1. A molecule comprising a module (A) capable of binding to a capsid of AAV (adeno-associated virus) ("AAV capsid-binding property") and a module (B) capable of binding to a target receptor ("target receptor-binding property"), and capable of simultaneously binding to the capsid of AAV and the target receptor, wherein
the module (A) is 1) an AAV capsid-binding peptide, or 2) a scaffold protein having a structure in which an AAV capsid-binding peptide is grafted ("the molecule").

2. The molecule according to claim 1, wherein the module (B) is 1) an anti-target receptor antibody, 2) a target receptor-binding peptide, or 3) a scaffold protein having a structure in which a target receptor-binding peptide is grafted.

3. The molecule according to claim 1 or 2, which is an anti-target receptor antibody having a structure in which an AAV capsid-binding peptide is grafted.

4. The molecule according to claim 1 or 2, which is a protein to which a scaffold protein having a structure in which an AAV capsid-binding peptide is grafted and an anti-target receptor antibody are linked.

5. The molecule according to claim 1 or 2, which is a protein to which a scaffold protein having a structure in which an AAV capsid-binding peptide is grafted and a scaffold protein having a structure in which a target receptor-binding peptide is grafted are linked.

6. The molecule according to claim 1 or 2, which is a scaffold protein having a structure in which an AAV capsid-binding peptide and a target receptor-binding peptide are grafted.

7. The molecule according to claim 1 or 2, which is an anti-target receptor antibody having an amino acid sequence incorporating all or part of an AAV capsid-binding peptide sequence,
which antibody is composed of an anti-target receptor antibody having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.

8. The molecule according to claim 1 or 2, which is a protein to which
an anti-target receptor antibody and an AAV capsid-binding scaffold protein having an amino acid sequence incorporating all or part of an AAV capsid-binding peptide sequence are linked,
which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure.

9. The molecule according to claim 1 or 2, which is a protein to which
an AAV capsid-binding scaffold protein having an amino acid sequence incorporating all or part of an AAV capsid-binding peptide sequence, which AAV capsid-binding scaffold protein is composed of a scaffold protein having at least one loop structure wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and
a target receptor-binding scaffold protein having an amino acid sequence incorporating all or part of a target receptor-binding peptide sequence, which target receptor-binding scaffold protein is composed of a scaffold protein having at least one loop structure wherein all or part of an amino acid sequence constituting the at least one loop structure is replaced with all or part of the target receptor-binding peptide sequence, or all or part of the target receptor-binding peptide sequence is inserted into an amino acid sequence constituting the at least one loop structure
are linked.

10. The molecule according to claim 1 or 2, which is a protein having an amino acid sequence incorporating all or part of each of an AAV capsid-binding peptide sequence and a target receptor-binding peptide sequence, which protein is composed of a scaffold protein having two or more loop structures wherein all or part of an amino acid sequence constituting the at least one loop structure is replaced with all or part of the AAV capsid-binding peptide sequence, or all or part of the AAV capsid-binding peptide sequence is inserted into an amino acid sequence constituting the at least one loop structure, and wherein all or part of an amino acid sequence constituting at least one loop structure different from the above-mentioned loop structure is replaced with all or part of the target receptor-binding peptide sequence, or all or part of the target receptor-binding peptide sequence is inserted into an amino acid sequence constituting the at least one loop structure different from the above-mentioned loop structure.

11. The molecule according to claim 1 according to claim 2, which is a protein in which a biotinylated AAV capsid-binding peptide and a biotinylated target receptor-binding peptide are linked via a protein having two or more biotin-binding sites.

12. The molecule according to claim 1 or 2, which is a protein in which a biotinylated AAV capsid-binding peptide and a biotinylated anti-target receptor antibody are linked via a protein having two or more biotin-binding sites.

13. The molecule according to claim 1 or 2, wherein the AAV capsid-binding peptide is a full sequence or a partial sequence constituting a cyclic peptide ("AAV capsid-binding cyclic peptide") that contains an AAV capsid-binding amino acid sequence in the peptide sequence.

14. The molecule according to claim 13, wherein the AAV capsid-binding cyclic peptide is a cyclic peptide having a cyclic structure formed via a crosslinking bond connecting one amino acid to another amino acid in the peptide sequence.

15. The molecule according to claim 13, wherein the AAV capsid-binding cyclic peptide has a cyclic structure comprising 4 to 30 amino acids.

16. The molecule according to claim 14, wherein the crosslinked structure comprises a thioether bond or a disulfide bond.

17. The molecule according to claim 13, wherein the AAV capsid-binding cyclic peptide has a cyclic structure formed via a crosslinking bond connecting an amino acid having a Functional Group 1 ("ring-forming amino acid 1") shown in Functional Group groups (A) to (E) in the following Table 1, with an amino acid having the corresponding Functional Group 2 ("ring-forming amino acid 2").
**[Table 1]**
| | **Functional Group 1** | **Functional Group 2** |
|---|---|---|
| (A) | 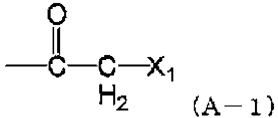 | HS- (A-2) |
| (B) | -C≡C-H (B- 1) | N₃- (B-2) |
| (C) | -Ar-CH₂NH₂ (C-1) | 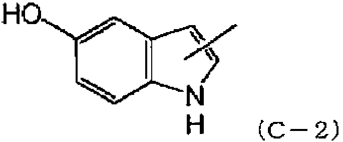 |
| (D) | -C≡C-CH₂-X₁ (D-1) | HS- (D-2) |
| (E) | -Ar-CH₂-X₁ (E - 1) | HS- (E-2) |
| | | |
|---|---|---|
| wherein X₁ is a leaving group, and Ar is an aromatic ring optionally having substituent(s). | | |

18. The molecule according to claim 17, wherein the ring-forming amino acid 1 is an amino acid having a Functional Group (A-1) described in the aforementioned Table 1, the ring-forming amino acid 2 is an amino acid having the corresponding Functional Group (A-2), and the cyclic structure comprises an - N-CO-CH₂-S- structure formed between the both amino acids.

19. The molecule according to claim 17, wherein N in the -N-CO-CH₂-S- structure is derived from an amino group of the ring-forming amino acid 1 selected from alanine, tyrosine, phenylalanine, tryptophan, ornithine, lysine, diaminopropionic acid, and diaminobutyric acid.

20. The molecule according to claim 17, wherein S in the -N-CO-CH₂-S- structure is derived from a thiol group of the ring-forming amino acid 2 selected from cysteine, homo cysteine, mercaptonorvaline, mercaptonorleucine, 2-amino-7-mercaptoheptanoic acid, and 2-amino-8-mercaptooctanoic acid.

21. The molecule according to claim 13, wherein the AAV capsid-binding cyclic peptide is a cyclic peptide comprising, in the peptide sequence, an ("AAV9 capsid-binding") amino acid sequence capable of binding to a capsid of AAV9 (adeno-associated virus serotype 9) ("AAV9 capsid-binding cyclic peptide").

22. The molecule according to claim 21, wherein the AAV9 capsid-binding cyclic peptide has a cyclic structure formed via
1) a crosslinking bond connecting one amino acid located at one end of the AAV9 capsid-binding amino acid sequence or located outside that end to another amino acid located at the other end of the AAV9 capsid-binding amino acid sequence or located outside that end, or
2) a crosslinking bond connecting one amino acid located at one end of the AAV9 capsid-binding amino acid sequence or located outside that end to another amino acid located within the AAV9 capsid-binding amino acid sequence.

23. The molecule according to claim 22, wherein the AAV9 capsid-binding amino acid sequence is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn.

24. The molecule according to claim 23, wherein the core motif sequence (II) is
(1) the amino acid sequence shown in Tyr-Ser-Lys-Ser-Val-Cys-Leu-Ala-Gln-Asp (SEQ ID NO: 2), or
an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.

25. The molecule according to any one of claims 21 to 24, wherein the active structure of the AAV9 capsid-binding cyclic peptide is selected from cyclic peptides represented by the following formula (Ia), the formula (Ib), and the formula (Ic): 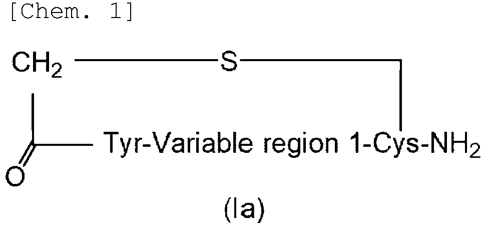
wherein Variable region 1 is an AAV9-binding amino acid sequence selected from the amino acid sequences listed in the following Table 2, and
S is S derived from the thiol group of Cys
**[Table 2]**
| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

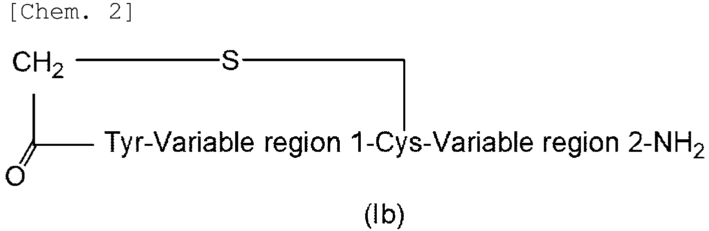
wherein Variable region 1 is an AAV9-binding amino acid sequence selected from the amino acid sequences listed in the following Table 3,
Variable region 2 is an AAV9-binding amino acid sequence selected from the amino acid sequences listed in the following Table 4, and
S is S derived from the thiol group of Cys
**[Table 3]**
| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |
wherein an amino acid sequence of Variable region 1 in Table 3 is preferably Ser Lys Ser Val (SKSV),
**[Table 4]**
| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

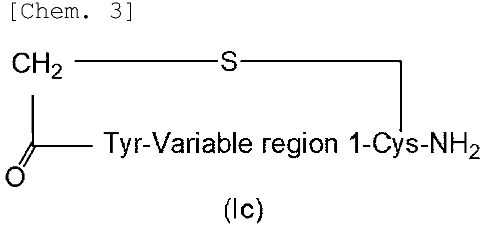
wherein Variable region 1 is an AAV9-binding amino acid sequence selected from the amino acid sequences listed in the following Table 5, and
S is S derived from the thiol group of Cys
**[Table 5]**
| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |
.

26. The molecule according to claim 1 or 2, wherein the AAV capsid-binding peptide is core motif sequence (I) represented by the following:
(1) an amino acid sequence shown in Tyr-Thr-Tyr-Arg-Thr-Thr-Ile-Pro-His-Glu-Tyr-Leu-Ala (SEQ ID NO: 1), or
(2) an amino acid sequence having 80% or more identity to the amino acid sequence shown in SEQ ID NO: 1 and having AAV9 capsid-binding property; or
core motif sequence (II) represented by the following:
an amino acid sequence shown by the formula: Xaa1-Ser-Lys-Ser-Xaa2-Xaa3-Leu-Ala-Gln-Xaa4
wherein Xaa1 is Phe or Tyr,
Xaa2 is Leu, Val, Ile, or Phe,
Xaa3 is any amino acid, and
Xaa4 is Gln, Asp, Glu, or Asn, or
a sequence comprising the sequence.

27. The molecule according to claim 26, wherein the core motif sequence (II) is
(1) the amino acid sequence shown in Tyr-Ser-Lys-Ser-Val-Cys-Leu-Ala-Gln-Asp (SEQ ID NO: 2), or
an amino acid sequence having 70% or more identity to the amino acid sequence shown in SEQ ID NO: 2 and having AAV9 capsid-binding property, or
(2) the amino acid sequence shown in Phe-Ser-Lys-Ser-Leu-His-Leu-Ala-Gln-Glu (SEQ ID NO: 3), or
an amino acid sequence having 70% or more identity to the amino acid sequence shown in SEQ ID NO: 3 and having AAV9 capsid-binding property.

28. The molecule according to claim 1 or 2, wherein the AAV capsid-binding peptide is a full sequence or a partial sequence constituting the cyclic peptide represented by the following formula (Ia), the formula (Ib), or the formula (Ic) 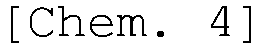 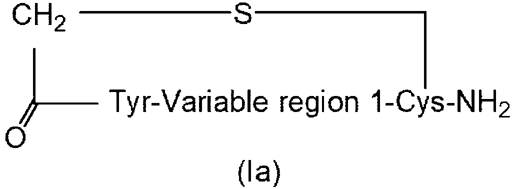
wherein Variable region 1 is an AAV9-binding amino acid sequence selected from the amino acid sequences listed in the following Table 6, and
S is S derived from the thiol group of Cys
**[Table 6]**
| | amino acid sequence (including single letter notation) | SEQ ID NO: |
|---|---|---|
| 1-1 | Thr Tyr Arg Thr Thr Ile Pro His Glu Tyr Leu Ala(TYRTTIPHEYLA) | 4 |
| 1-2 | Thr Tyr Arg Thr Thr Ile Pro Asn Glu Tyr Leu Ala(TYRTTIPNEYLA) | 5 |
| 1-3 | Thr Tyr Arg Thr Thr Ile His His Glu Tyr Leu Ala(TYRTTIHHEYLA) | 6 |
| 1-4 | Thr Tyr Arg Thr Thr Ile Pro His Glu Tyr Ile Ala(TYRTTIPHEYIA) | 7 |
| 1-5 | Thr Tyr Arg Thr Thr Ile Thr His Glu Tyr Leu Ala(TYRTTITHEYLA) | 8 |

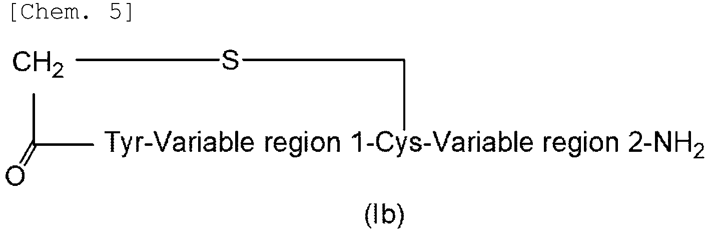
wherein Variable region 1 is an AAV9-binding amino acid sequence selected from the amino acid sequences listed in the following Table 7,
Variable region 2 is an AAV9-binding amino acid sequence selected from the amino acid sequences listed in the following Table 8, and
S is S derived from the thiol group of Cys
**[Table 7]**
| | amino acid sequence (including single letter notation) | SEQ ID NO: |
|---|---|---|
| 2-1 | Ser Lys Ser Val (SKSV) | 9 |
| 2-2 | Thr-Lys-Ser-Thr (TKST) | 10 |
| 2-3 | Ser-Lys-Ser-Ile (SKSI) | 11 |
| 2-4 | Ser-Lys-Ser-Ala (SKSA) | 12 |
| 2-5 | Ser-Lys-Arg-Val (SKRV) | 13 |
wherein the amino acid sequence of Variable region 1 in Table 7 is preferably Ser Lys Ser Val (SKSV),
**[Table 8]**
| | amino acid sequence (including single letter notation) | SEQ ID NO: |
|---|---|---|
| 2-A | Leu Ala Gln Asp Trp Glu Tyr Lys Trp (LAQDWEYKW) | 14 |
| 2-B | Leu Ala Gln Asp Trp Glu Tyr Lys (LAQDWEYK) | 15 |
| 2-C | Leu Ala Lys Asp Trp Glu Tyr Lys Trp (LAKDWEYKW) | 16 |
| 2-D | Leu Ala Gln Asp Trp Val Tyr Lys Tyr (LAQDWVYKY) | 17 |
| 2-E | Leu Ala Gln Asp Trp Lys Tyr Lys Trp (LAQDWKYKW) | 18 |

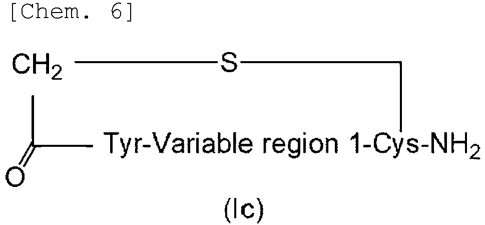
wherein Variable region 1 is an AAV9-binding amino acid sequence selected from the amino acid sequences listed in the following Table 9, and
S is S derived from the thiol group of Cys
**[Table 9]**
| | amino acid sequence (including single letter notation) | SEQ ID NO: |
|---|---|---|
| 3-1 | Arg Phe Ser Lys Ser Leu His Leu Ala Gln Gln Tyr Gln Arg Ile (RFSKSLHLAQQYQRI) | 19 |
| 3-2 | Arg Phe Ser Lys Ser Leu His Leu Ala Gln Glu Tyr Gln Arg Ile (RFSKSLHLAQEYQRI) | 20 |
| 3-3 | Arg Phe Ser Lys Ser Leu His Leu Ala Gln Gln Tyr Gln Gln Ile (RFSKSLHLAQQYQQI) | 21 |
| 3-4 | Arg Phe Ser Lys Ser Leu His Leu Ala Gln Gln Tyr Lys Arg Ile (RFSKSLHLAQQYKRI) | 22 |
| 3-5 | Arg Phe Ser Lys Ser Leu His Leu Ala Gln Gln Tyr Gln Leu Ile (RFSKSLHLAQQYQLI) | 23 |

29. The molecule according to claim 1 or 2, wherein the target receptor-binding peptide is a full sequence or a partial sequence constituting a cyclic peptide comprising a target receptor-binding amino acid sequence in the peptide sequence ("target receptor-binding cyclic peptide").

30. The molecule according to claim 29, wherein the target receptor-binding cyclic peptide is a cyclic peptide having a cyclic structure formed via a crosslinking bond connecting one amino acid to another amino acid in the peptide sequence.

31. The molecule according to claim 1 or 2, that binds to AAV via module (A), for attenuating the non-specific infectivity of AAV, while simultaneously increasing the specific infectivity for the receptor that is the target of module (B).

32. An AAV vector comprising a complex of the molecule according to claim 1 or 2 and an AAV having a wild-type capsid, which complex is obtained by mixing the molecule and the AAV.

33. The AAV vector according to claim 32, in which a foreign molecule is loaded onto the AAV.

34. The AAV vector according to claim 32, for infecting cells that present a target receptor with AAV.

35. The AAV vector according to claim 33, for introducing a foreign molecule into cells that present a target receptor.
